# EUROPEAN PATENT APPLICATION

(11) **EP 2 161 577 A1**
(43) Date of publication of application: **10.03.2010**
(21) Application number: 08163428.9
(22) Date of filing: 01.09.2008
(51) Int. Cl.: G01N 33/574, C07K 14/47, G01N 33/68

(54) **ANLN protein**

(71) Applicant: Atlas Antibodies AB, 106 91 Stockholm (SE)
(72) Inventor: Uhlén, Mathias, 182 79 Stocksund (SE); Pontén, Fredrik, 752 37 Uppsala (SE); Jirström, Karin, 216 18 Limhamn (SE)
(74) Representative: Mattsson, Niklas

(57) **Abstract**

The present invention provides methods, uses and means for breast cancer prognostics and treatment prediction. Provided methods comprises the steps of: providing a sample earlier obtained from a breast cancer subject; evaluating the amount of ANLN protein present in at least part of said sample, and determining a sample value corresponding to said amount; comparing the sample value with a reference value; and, if said sample value is higher than said reference value, concluding that the subject is not likely to benefit from an endocrine treatment or that a prognosis for said subject is worse than a reference prognosis associated with the reference value.

SEQUENCE LISTING

<110>  Atlas Antibodies

<120>  ANLN protein

<130>  21037817

<160>  3

<170>  PatentIn version 3.3

<210>  1
<211>  136
<212>  PRT
<213>  Homo sapiens

<400>  1

Ile Val Lys Ser Thr Leu Ser Gln Thr Val Pro Ser Lys Gly Glu Leu
1               5                   10                  15

Ser Arg Glu Ile Cys Leu Gln Ser Gln Ser Lys Asp Lys Ser Thr Thr
            20                  25                  30

Pro Gly Gly Thr Gly Ile Lys Pro Phe Leu Glu Arg Phe Gly Glu Arg
        35                  40                  45

Cys Gln Glu His Ser Lys Glu Ser Pro Ala Arg Ser Thr Pro His Arg
    50                  55                  60

Thr Pro Ile Ile Thr Pro Asn Thr Lys Ala Ile Gln Glu Arg Leu Phe
65                  70                  75                  80

Lys Gln Asp Thr Ser Ser Ser Thr Thr His Leu Ala Gln Gln Leu Lys
                85                  90                  95

Gln Glu Arg Gln Lys Glu Leu Ala Cys Leu Arg Gly Arg Phe Asp Lys
            100                 105                 110

Gly Asn Ile Trp Ser Ala Glu Lys Gly Gly Asn Ser Lys Ser Lys Gln
        115                 120                 125

Leu Glu Thr Lys Gln Glu Thr His
    130                 135

<210>  2
<211>  1124
<212>  PRT
<213>  Homo sapiens

<400>  2

<210> 3
<211> 4768
<212> DNA
<213> Homo sapiens

<400> 3
ctcggcgctg aaattcaaat ttgaacggct gcagaggccg agtccgtcac tggaagccga 60

gaggagagga cagctggttg tgggagagtt cccccgcctc agactcctgg ttttttccag 120

gagacacact gagctgagac tcacttttct cttcctgaat ttgaaccacc gtttccatcg 180

tctcgtagtc cgacgcctgg ggcgatggat ccgtttacgg agaaactgct ggagcgaacc 240

cgtgccaggc gagagaatct tcagagaaaa atggctgaga ggcccacagc agctccaagg 300

tctatgactc atgctaagcg agctagacag ccactttcag aagcaagtaa ccagcagccc 360

ctctctggtg gtgaagagaa atcttgtaca aaaccatcgc catcaaaaaa acgctgttct 420

gacaacactg aagtagaagt ttctaacttg gaaaataaac aaccagttga gtcgacatct 480

gcaaaatctt gttctccaag tcctgtgtct cctcaggtgc agccacaagc agcagatacc 540

atcagtgatt ctgttgctgt cccggcatca ctgctgggca tgaggagagg gctgaactca 600

agattggaag caactgcagc ctcctcagtt aaaacacgta tgcaaaaact tgcagagcaa 660

cggcgccgtt gggataatga tgatatgaca gatgacattc ctgaaagctc actcttctca 720

ccaatgccat cagaggaaaa ggctgcttcc cctcccagac ctctgctttc aaatgcctcg 780

gcaactccag ttggcagaag gggccgtctg gccaatcttg ctgcaactat ttgctcctgg 840

gaagatgatg taaatcactc atttgcaaaa caaaacagtg tacaagaaca gcctggtacc 900

gcttgtttat ccaaattttc ctctgcaagt ggagcatctg ctaggatcaa tagcagcagt 960

gttaagcagg aagctacatt ctgttcccaa agggatggcg atgcctcttt gaataaagcc 1020

ctatcctcaa gtgctgatga tgcgtctttg gttaatgcct caatttccag ctctgtgaaa 1080

gctacttctc cagtgaaatc tactacatct atcactgatg ctaaaagttg tgagggacaa 1140

aatcctgagc tacttccaaa aactcctatt agtcctctga aaacgggggt atcgaaacca 1200

attgtgaagt caactttatc ccagacagtt ccatccaagg gagaattaag tagagaaatt 1260

tgtctgcaat ctcaatctaa agacaaatct acgacaccag gaggaacagg aattaagcct 1320

ttcctggaac gctttggaga gcgttgtcaa gaacatagca aagaaagtcc agctcgtagc 1380

acaccccaca gaacccccat tattactcca aatacaaagg ccatccaaga aagattattc 1440

aagcaagaca catcttcatc tactacccat ttagcacaac agctcaagca ggaacgtcaa 1500

aaagaactag catgtcttcg tggccgattt gacaagggca atatatggag tgcagaaaaa 1560

ggcggaaact caaaaagcaa acaactagaa accaaacagg aaactcactg tcagagcact 1620

cccctcaaaa aacaccaagg tgtttcaaaa actcagtcac ttccagtaac agaaaaggtg 1680

accgaaaacc agataccagc caaaaattct agtacagaac ctaaaggttt cactgaatgc 1740

gaaatgacga aatctagccc tttgaaaata acattgtttt tagaagagga caaatcctta 1800

aaagtaacat cagacccaaa ggttgagcag aaaattgaag tgatacgtga aattgagatg 1860

agtgtggatg atgatgatat caatagttcg aaagtaatta atgacctctt cagtgatgtc 1920
ctagaggaag gtgaactaga tatggagaag agccaagagg agatggatca agcattagca 1980
gaaagcagcg aagaacagga agatgcactg aatatctcct caatgtcttt acttgcacca 2040
ttggcacaaa cagttggtgt ggtaagtcca gagagtttag tgtccacacc tagactggaa 2100
ttgaaagaca ccagcagaag tgatgaaagt ccaaaaccag gaaaattcca aagaactcgt 2160
gtccctcgag ctgaatctgg tgatagcctt ggttctgaag atcgtgatct tctttacagc 2220
attgatgcat atagatctca aagattcaaa gaaacagaac gtccatcaat aaagcaggtg 2280
attgttcgga aggaagatgt tacttcaaaa ctggatgaaa aaaataatgc ctttccttgt 2340
caagttaata tcaaacagaa aatgcaggaa ctcaataacg aaataaatat gcaacagaca 2400
gtgatctatc aagctagcca ggctcttaac tgctgtgttg atgaagaaca tggaaaaggg 2460
tccctagaag aagctgaagc agaaagactt cttctaattg caactgggaa gagaacactt 2520
ttgattgatg aattgaataa attgaagaac gaaggacctc agaggaagaa taaggctagt 2580
ccccaaagtg aatttatgcc atccaaagga tcagttactt tgtcagaaat ccgcttgcct 2640
ctaaaagcag attttgtctg cagtacggtt cagaaaccag atgcagcaaa ttactattac 2700
ttaattatac taaaagcagg agctgaaaat atggtagcca caccattagc aagtacttca 2760
aactctctta acggtgatgc tctgacattc actactacat ttactctgca agatgtatcc 2820
aatgactttg aaataaatat tgaagtttac agcttggtgc aaaagaaaga tccctcaggc 2880
cttgataaga agaaaaaaac atccaagtcc aaggctatta ctccaaagcg actcctcaca 2940
tctataacca caaaaagcaa cattcattct tcagtcatgg ccagtccagg aggtcttagt 3000
gctgtgcgaa ccagcaactt cgcccttgtt ggatcttaca cattatcatt gtcttcagta 3060
ggaaatacta agtttgttct ggacaaggtc ccctttttat cttctttgga aggtcatatt 3120
tatttaaaaa taaaatgtca agtgaattcc agtgttgaag aaagaggttt tctaaccata 3180
tttgaagatg ttagtggttt tggtgcctgg catcgaagat ggtgtgttct ttctggaaac 3240
tgtatatctt attggactta tccagatgat gagaaacgca agaatcccat aggaaggata 3300
aatctggcta attgtaccag tcgtcagata gaaccagcca acagagaatt ttgtgcaaga 3360
cgcaacactt ttgaattaat tactgtccga ccacaaagag aagatgaccg agagactctt 3420
gtcagccaat gcagggacac actctgtgtt accaagaact ggctgtctgc agatactaaa 3480
gaagagcggg atctctggat gcaaaaactc aatcaagttc ttgttgatat tcgcctctgg 3540
caacctgatg cttgctacaa acctattgga aagccttaaa ccgggaaatt tccatgctat 3600
ctagaggttt ttgatgtcat cttaagaaac acacttaaga gcatcagatt tactgattgc 3660
attttatgct ttaagtacga aagggtttgt gccaatattc actacgtatt atgcagtatt 3720
tatatctttt gtatgtaaaa ctttaactga tttctgtcat tcatcaatga gtagaagtaa 3780
atacattata gttgattttg ctaaatctta atttaaaagc ctcattttcc tagaaatcta 3840
attattcagt tattcatgac aatatttttt taaaagtaag aaattctgag ttgtcttctt 3900
ggagctgtag gtcttgaagc agcaacgtct ttcaggggtt ggagacagaa acccattctc 3960

caatctcagt agttttttcg aaaggctgtg atcatttatt gatcgtgata tgacttgtta 4020
ctagggtact gaaaaaaatg tctaaggcct ttacagaaac atttttagta atgaggatga 4080
gaactttttc aaatagcaaa tatatattgg cttaaagcat gaggctgtct tcagaaaagt 4140
gatgtggaca taggaggcaa tgtgtgagac ttgggggttc aatattttat atagaagagt 4200
taataagcac atggtttaca tttactcagc tactatatat gcagtgtggt gcacattttc 4260
acagaattct ggcttcatta agatcattat ttttgctgcg tagcttacag acttagcata 4320
ttagtttttt ctactcctac aagtgtaaat tgaaaaatct ttatattaaa aaagtaaact 4380
gttatgaagc tgctatgtac taataatact ttgcttgcca aagtgtttgg gttttgttgt 4440
tgtttgtttg tttgtttgtt tttggttcat gaacaacagt gtctagaaac ccattttgaa 4500
agtggaaaat tattaagtca cctatcacct ttaaacgcct ttttttaaaa ttataaaata 4560
ttgtaaagca gggtctcaac ttttaaatac actttgaact tcttctctga attattaaag 4620
ttctttatga cctcatttat aaacactaaa ttctgtcacc tcctgtcatt ttatttttta 4680
ttcattcaaa tgtatttttt cttgtgcata ttataaaaat atattttatg agctcttact 4740
caaataaata cctgtaaatg tctaaagg 4768

## Description

### Field of the Invention

The present invention relates to the field of breast cancer. In particular, it relates to prognostics, treatment and treatment prediction within that field.

### Background

### Cancer

Cancer is one of the most common causes of disease and death in the western world. In general, incidence rates increase with age for most forms of cancer. As human populations continue to live longer, due to an increase of the general health status, cancer may affect an increasing number of individuals. The cause of most common cancer types is still largely unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. A malignant tumor does not only necessarily consist of the transformed tumor cells themselves but also surrounding normal cells which act as a supportive stroma. This recruited cancer stroma consists of connective tissue, blood vessels and various other normal cells, e.g., inflammatory cells, which act in concert to supply the transformed tumor cells with signals necessary for continued tumor growth.

The most common forms of cancer arise in somatic cells and are predominantly of epithelial origin, e.g., prostate, breast, colon, urothelial and skin, followed by cancers originating from the hematopoetic lineage, e.g., leukemia and lymphoma, neuroectoderm, e.g., malignant gliomas, and soft tissue tumors, e.g., sarcomas.

### Cancer diagnostics and prognostics

Microscopic evaluation of a tissue section taken from a tumor remains the golden standard for determining a diagnosis of cancer. For example, for microscopic diagnosis, biopsy material from suspected tumors is collected and examined under the microscope. To obtain a firm diagnosis, the tumor tissue is fixated in formalin, histo-processed and paraffin embedded. From the resulting paraffin block, tissue sections can be produced and stained using both histochemical, i.e., hematoxylin-eosin staining, and immunohistochemical methods. The surgical specimen is then evaluated with pathology techniques, including gross and microscopic analysis. This analysis often forms the basis for assigning a specific diagnosis, i.e., classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e., how far the tumor has invaded and imposed growth into surrounding normal tissues, whereas the N stage and M stage describe how the tumor has developed into metastasis, with the N stage describing spread of tumor to lymph nodes and the M stage describing growth of tumor in other distant organs. Early stages include: TO-1, NO, MO, representing localized tumors with negative lymph nodes. More advanced stages include: T2-4, NO, MO, localized tumors with more widespread growth and T1-4, N1-3, MO, tumors that have metastasized to lymph nodes and T1-4, N1-3, M1, tumors with a metastasis detected in a distant organ. Staging of tumors is often based on several forms of examination, including surgical, radiological and histopathological analyses. In addition to staging, there is also a classification system to grade the level of malignancy for most tumor types. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and the Nottingham Histological Grade (NHG) grading for breast carcinomas.

Accurate staging and grading is crucial for a correct diagnosis and may provide an instrument to predict a prognosis. The diagnostic and prognostic information for a specific tumor subsequently determines an adequate therapeutic strategy for a given cancer patient. A commonly used method, in addition to histochemical staining of tissue sections, to obtain more information regarding a tumor is immunohistochemical staining. IHC allows for the detection of protein expression patterns in tissues and cells using specific antibodies. The use of IHC in clinical diagnostics allows for the detection of immunoreactivity in different cell populations, in addition to the information regarding tissue architecture and cellular morphology that is assessed from the histochemically stained tumor tissue section. IHC can be involved in supporting the accurate diagnosis, including staging and grading, of a primary tumor as well as in the diagnostics of metastases of unknown origin. The most commonly used antibodies in clinical practice today include antibodies against cell type "specific" proteins, e.g., PSA (prostate), MelanA (melanocytes) and Thyroglobulin (thyroid gland), and antibodies recognizing intermediate filaments (epithelial, mesenchymal, glial), cluster of differentiation (CD) antigens (hematopoetic, sub-classification of lympoid cells) and markers of malignant potential, e.g., Ki67 (proliferation), p53 (commonly mutated tumor suppressor gene) and HER-2 (growth factor receptor).

Aside from IHC, the use of *in situ* hybridization for detecting gene amplification and gene sequencing for mutation analysis are evolving technologies within cancer diagnostics. In addition, global analysis of transcripts, proteins or metabolites all add relevant information. However, most of these analyses still represent basic research and have yet to be evaluated and standardized for the use in clinical medicine.

In order for physicians to give a cancer patient the right type of treatment as early as possible, the provision of new molecular markers that allow for more accurate stratification of cancer patients into different risk categories is of interest. In summary, there exists a need for new means to advance prognostics and staging of cancer.

### Breast cancer

Breast cancer is the second most common form of cancer worldwide and by far the most frequent cancer of women. Data from the GLOBOCAN 2002 database presented by Parkin *et al*. reveal 1.15 million new cases in 2002 and 0.41 million deaths during the same period (Parkin DM et al. (2005) CA Cancer J Clin 55, 74-108). If detected at an early stage, the prognosis is relatively good for a patient living in a developed country, with a general five-year survival rate of 73%, compared to 57% in a developing country. The incidence is slowly increasing and about one in every nine women in the developed world is believed to get breast cancer in her lifetime. Although lifestyle changes related to female steroid hormones, including exposure to exogenous hormones, affect the risk of developing breast cancer, these factors only make up for a small fraction of the etiology, and the benefit of preventive manipulation is believed to be low. The decreased mortality is mainly due to earlier detection by mammography screening and the use of modern adjuvant systemic treatment.

### Treatment of breast cancer

Since its introduction in the late seventies, breast-conserving therapy, combining breast conserving surgery and postoperative radiotherapy, has become the primary treatment of choice in women where radical removal of the tumor can be combined with a good cosmetic result. Mastectomy is still preferable in some patients, i.e., women with small breasts, large tumors (> 4 cm) or multifocal/multicentric disease.

Axillary dissection is primarily performed for diagnostic purposes and removal of at least 10 lymph nodes gives a good staging guidance with 97-98% sensitivity (Axelsson CK et al. (1992) Eur J Cancer 28A:1415-8; Recht A and Houlihan MJ (1995) Cancer 6(9):1491-1512). However, the next step towards minimal surgery in the treatment of primary cancer has been the introduction of the sentinel node biopsy technique with mapping of axillary lymph nodes instead of axillary lymph node clearance, which is associated with a high complication rate. This technique was introduced as a consequence of the knowledge that most of the lymphatic drainage to the axilla from the breast initially passes through one (or a few) lymph node(s) - the sentinel node(s) - supporting that analysis of this lymph node may be a sufficient indicator of axillary node status (Veronesi U et al. (2003) New Engl J Med 349(6): 546-53.)

The concept of breast cancer as a systemic disease, i.e., the presence of disseminating micro-metastases at the time of diagnosis that may explain treatment failure after locoregional therapy, paved the way for adjuvant randomized trials in the 1970s, including endocrine therapy and chemotherapy. Adjuvant polychemotherapy is standard treatment for hormone-receptor negative patients with high risk of recurrence, irrespective of nodal status. A beneficial effect on both overall- and relapse-free survival has been demonstrated, especially in premenopausal patients (EBCTCG (1998) Lancet 352(9132): 930-42). For patients with hormone-responsive disease, i.e., estrogen receptor (ER) and/or progesterone receptor (PR) positive disease, adjuvant polychemotherapy has been delivered in combination with endocrine therapy as sequential chemo-endocrine therapy. Also, adjuvant chemotherapy generally induces amenorrhea, causing a secondary endocrine effect in addition to the cytotoxic (Pagani O et al. (1998) Eur J Cancer 34(5):632-40).

Endocrine therapy has been recommended for patients with hormone receptor positive tumors irrespective of age, stage and menopausal status.

In hormone-responsive premenopausal patients, ovarian ablation by surgery or irradiation, or ovarian suppression by LHRH agonists are efficient adjuvant treatment modalities (Emens LA and Davidson NA (2003) Clin Ca Res (1 Pt 2): 468S-94S). In postmenopausal patients, ovarian ablation has no place, since the primary source of estrogen is not from ovarian synthesis but from the conversion of androstenedione to estrone and estradiol in peripheral tissues including the breast.

Tamoxifen is a selective estrogen receptor modulator (SERM) with an agonistic effect on the ER, making it a suitable treatment for advanced breast cancer in both pre- and postmenopausal women. Studies have shown that five years of tamoxifen as adjuvant treatment after primary surgery reduces the breast cancer mortality in patients with ER positive (ER+) tumors, irrespective of lymph node status (EBCTCG (1998) Lancet 351 (9114):1451-67). While tamoxifen has a protective effect against cardiovascular disease, the risk of developing endometrial cancer is increased, due to an agonistic effect on the ER in the endometrium (EBCTCG (2005) Lancet 365(9472):1687-717)

Aromatase inhibitors (Als) function by inhibiting aromatase, the enzyme converting androgens into estrogens. Als are not suitable for treatment of premenopausal women, as it stimulates the ovaries to an increased androgen production through the hypothalamus and pituitary gland. Als can be given as adjuvant treatment to postmenopausal women, either alone or following tamoxifen treatment and they have been shown to significantly reduce the mortality, possibly even more if given alone (Howell A et al. (1995) Lancet 345(8941):29-30; Ellis MJ and Rigden CE (2006) Curr Med Res Opin 22(12):2479-87; Coates AS et al. (2007) J Clin Oncol 25(5):486-92). However, this therapy is relatively new and the long-term side effects are not yet fully known (Buzdar A et al. (2006) Lancet Oncol 7(8):633-43), but the most important are cardiovascular complications and osteoporosis.

Newly developed pure anti-estrogens such as fulvestrant, which completely blocks the ER, are currently only used in advanced breast cancer and not in the adjuvant setting (Rutqvist LE (2004) Best Pract Res Clin Endocrinol Metab 18(1): 81-95).

Adjuvant endocrine therapy is believed to have no place in hormone receptor negative breast cancer, although some studies indicate that some ER negative (ER-), i.e., ERα negative (ERα-), tumors respond to tamoxifen treatment (EBCTCG (1998) Lancet 351:1451-1467)

The HER2/neu gene is overexpressed in about 20% of all, and in up to 70% of lowly differentiated, breast cancers (Berger MS et al. (1988) Cancer Res 48(5):1238-43; Borg A et al. (1990) Cancer Res 50(14): 4332-7). Patients with HER2 overexpressing tumors may benefit from treatment with the monoclonal antibody trastuzumab. Experimental data support a relationship between HER2 overexpression and resistance to endocrine treatment (Shou J et al. (2004) J Natl Cancer Inst 96(12):926-35) while clinical data are not consistent (Borg A et al. (1994) Cancer Lett 81 (2):137-44, De Placido S et al. (2003) Clin Ca Res 9(3):1039-46, Rydén L et al. (2005) J Clin Oncol 23(21):4695-704).

### Breast cancer diagnostics

Morphologic criteria are still important in the establishment of a breast cancer diagnosis, both *in situ* and invasive cancer. Among invasive breast carcinomas, *invasive ductal carcinoma* is the most common tumor type (∼80 %) and *lobular carcinoma* is the second largest entity (∼10-15%). *Tubular* and *medullary* carcinomas are other distinct types with lower prevalence (WHO, Histological typing of breast tumors, in International histological classification of tumors no:2, 1981, WHO, Geneva).

### Breast cancer prognostics and treatment predictive factors

A correct histological classification of the tumor type may be of prognostic relevance, since certain subtypes, such as medullary carcinomas, in general have a more favorable prognosis. Nevertheless, assessment of the histological grade using the Nottingham Histological Grade (NHG) system is still a prognostic tool (Elston CW and Ellis lO (1991) 19(5):403-10; Sundquist M et al. (1999) Breast Cancer Res Treat 53(1):1-8).

The majority of breast cancers are hormone receptor responsive, i.e., express the ER and/or PR. The action of estrogen is mediated by the two receptors ERα and ERβ. ERα and PR are routinely assessed in order to select patients for endocrine therapy, and tumors with >10% nuclear positivity are considered positive. ERα is today considered a predictor of tamoxifen response. However, there are studies that indicate that PR positivity may even be a more powerful predictor of tamoxifen response than ERα. A study of premenopausal patients randomly treated with tamoxifen revealed that a high, >75% nuclear fraction, PR level was significantly correlated with an increased recurrence-free and overall survival, irrespectively of ERα status. At a lower PR level, <75%, no positive effect was observed (Stendahl M et al. (2006) Clin Cancer Res 12:4614-18). Yu *et al.* recently studied the predictive value of PR for adjuvant endocrine therapy, and found that older patients (≥ 60 years) with ERα+/PR+ tumors had a significantly longer disease free survival when treated with tamoxifen than patients that received no adjuvant treatment. In younger patients (<60 years), no significant effect was observed (Yu KD et al. (2007) The Breast 16:307-315). Interestingly, a report from the ATAC trial (postmenopausal women treated with arimidex, tamoxifen or in combination), showed that the recurrence rate was halved for anastrozole-treated patients with ERα+/PR-tumors over the follow-up period of 6 years, compared to patients treated with tamoxifen (Dowsett M et al. (2005) J Clin Oncol 23(30):7512-7).

The role of ERβ in breast cancer is not yet fully clarified, although recent studies implicate that ERβ-expression may be associated with a better tamoxifen response (Borgquist S et al, (2008) J Clin Pathol 61 (2):197-203), particularly in ERα- tumors (Gruvberger-Saal SK et al. (2007) Clin Cancer Res 13:1987-1994). However, determination of ERβ is today generally not considered clinically relevant.

A major problem to day is that 30-40% of the ERα positive (ERα+) patients do not respond to tamoxifen treatment (Riggins RB et al. (2007) Cancer Letters 1:1-24, Gruvberger-Saal SK et al. (2007) Clin Cancer Res 13:1987-1994), which results in unnecessary treatment. In addition, a fraction of the ERα- patients do respond to tamoxifen treatment, and the reason for that is currently not known. Gruvberger-Saal suggests that ERβ expression may be a positive predictor of tamoxifen response in ERα- patients (Gruvberger-Saal SK et al. (2007) Clin Cancer Res 13:1987-1994).

HER2 status is also assessed routinely, primarily by IHC and in cases with moderate expression, gene amplification status is determined by fluorescence in situ hybridization (FISH) analysis. Patients with a HER2 positive tumor may be treated with trastuzumab.

Breast cancer is a truly heterogeneous disease and despite the increasing understanding of its nature, the arsenal of available prognostic and treatment predictive markers is still not sufficient and some patients may therefore receive unnecessary treatment while others may get insufficient or even ineffective treatment. Additional molecular markers are needed in order to better define different subgroups of breast cancer and increase the options for tailored therapies.

### Endpoint analysis

Endpoint analysis is used to evaluate trials with adjuvant treatments for cancer as this gives information on how the patients respond to a certain therapy. Overall survival (OS) has been considered the standard primary endpoint. OS takes in to account time to death, irrespective of cause, e.g., if the death is due to cancer or not. Loss to follow-up is censored and regional recurrence, distant metastases, second primary breast cancers, and second other primary cancers are ignored.

To date, an increasing number of effective treatments available in many types of cancer have resulted in the need for surrogate endpoints to allow for a better evaluation of the effect of adjuvant treatments. Thus, the much longer follow-up required to demonstrate that adjuvant treatments improve OS is often complemented with other clinical endpoints that give an earlier indication on how successful the treatment is.

Endpoint analysis may also be useful for studies of a potential biomarker. In the present disclosure, the inventors show that the level of expression of a particular protein (the proposed biomarker) significantly correlates with prognoses. For these observations, primarily two surrogate endpoints are used, namely recurrence-free survival (RFS) and breast cancer-specific survival (BCSS). Analysis of RFS includes time to any event related to the same cancer, i.e., all cancer recurrences and deaths from the same cancer are events. Also distant, local and regional metastases as well as breast cancer specific death are considered. On the other hand, second primary same cancers and other primary cancers are ignored, as well as contralateral breast cancer. Deaths from other cancers, non-cancer-related deaths, treatment-related deaths, and loss to follow-up are censored observations. Breast cancer-specific survival (BCSS) includes time to death caused by breast cancer due to the original tumor. Both endpoints are relevant, since similarities or differences may reflect different tumor biological behaviors. Biomarkers associated with a locally aggressive behavior may for instance have greater impact on RFS than BCSS, while biomarkers associated with the development of distant metastases may be reflected in both RFS and BCSS.

### ANLN

The actin binding protein anillin (ANLN) is a subunit of microfilaments, one of the major components of the cytoskeleton. It is expressed in the majority of eukaryotic cells and has a role in basic cellular functions such as muscle contraction, cell motility, vesicle and organelle movement, cell division, cytokinesis and signaling.

The PCT publication WO 2005/001138 discloses measurements of the relative RNA expression of about 22000 genes from 124 patients. 7090 of these genes are selected based on their expression pattern. By means of hierarchical clustering, a cluster of 67 genes is obtained, and further, a patient group underexpressing all the 67 genes is associated with a better survival than a patient group overexpressing all the 67 genes. The ANLN gene is mentioned as one of the 67 genes, but is not studied individually. 4 other genes (DEEPEST, RACGAP1, ZNF145 and MS4A7) are identified as "strong prognostic factors individually". Protein expression is neither measured nor analyzed.

The PCT publication WO 2008/048193 relates to breast tumor grading and discloses global gene expression analysis by means of microarray experiments and statistical analysis. A list of 264 grade related genes, of which the ANLN gene is one, is disclosed. It is concluded that "the genetic essence of histological grade can be distilled down to the expression pattern of a mere 5 genes with powerful prognostic implications". However, the prognostic relevance of any individual genes is not shown. Further, protein expression is neither measured nor analyzed.

### Brief description

It is an object of some aspects of the present disclosure to provide means and methods useful in the establishment of a prognosis for a mammalian subject having a breast cancer. An object of other aspects is to provide means and methods useful in treatment prediction regarding such a subject.

The present invention is defined by the itemized listing of embodiments and the appending claims.

The following is a non-limiting and itemized listing of embodiments of the present disclosure, presented for the purpose of providing various features and combinations provided by the invention in certain of its aspects.

### ITEMS

1. Method for determining whether a mammalian subject having a breast cancer is likely to benefit from an endocrine treatment, comprising the steps of:
   a) providing a sample earlier obtained from said subject;
   b) evaluating the amount of ANLN protein present in at least part of said sample, and determining a sample value corresponding to said amount;
   c) comparing the sample value obtained in step b) with a reference value; and,
   if said sample value is higher than said reference value,
   d1) concluding that the subject is not likely to benefit from an endocrine treatment, or
      if said sample value is lower than or equal to said reference value,
   d2) concluding that the subject is likely to benefit from an endocrine treatment.
2. Non-treatment strategy method for a subject having a breast cancer, comprising:
   a) providing a sample earlier obtained from the subject;
   b) evaluating the amount of ANLN protein present in at least part of said sample, and determining a sample value corresponding to said evaluated amount;
   c) comparing the sample value obtained in step b) with a reference value;
      and, if said sample value is higher than said reference value,
   d) refraining from treating said subject with an endocrine treatment.
3. Method of treatment of a subject in need thereof, wherein said subject has a breast cancer, said method comprising the steps of:
   a) providing a sample from said subject;
   b) evaluating the amount of ANLN protein present in at least part of said sample, and determining a sample value corresponding to said amount;
   c) comparing the sample value obtained in step b) with a reference value; and,
   if said sample value is higher than said reference value,
   d1) treating said subject with a non-endocrine breast cancer treatment regimen, or
      if said sample value is lower than or equal to said reference value,
   d2) treating said subject with an endocrine treatment regimen.
4. Method according to item 3, wherein said non-endocrine breast cancer treatment regime is a chemotherapy and/or radiation therapy.
5. Method for determining whether a prognosis for a mammalian subject having a breast cancer is worse than a reference prognosis, comprising the steps of:
   a) providing a sample earlier obtained from said subject;
   b) evaluating the amount of ANLN protein present in at least part of said sample, and determining a sample value corresponding to said evaluated amount;
   c) comparing said sample value obtained in step b) with a reference value associated with said reference prognosis; and, if said sample value is higher than said reference value,
   d) concluding that said prognosis for said subject is worse than said reference prognosis.
6. Method according to item 5, wherein said subject has undergone, is undergoing and/or is scheduled for endocrine treatment.
7. Method according to any one of items 1-6, wherein said endocrine treatment is selected from a SERM treatment, an aromatase inhibitor treatment, and a steroidal estrogen receptor antagonist treatment.
8. Method according to any one of items 1-7, wherein said endocrine treatment is a SERM treatment and said SERM is selected from toremifene, raloxifene, droloxifene, arzoxifene and tamoxifen.
9. Method according to item 8, wherein said endocrine treatment is tamoxifen treatment.
10. Method of treatment of a subject in need thereof, wherein said subject is having a breast cancer, comprising the steps of:
   a) providing a sample from said subject;
   b) evaluating the amount of ANLN protein present in at least part of said sample, and determining a sample value corresponding to said evaluated amount;
   c) comparing said sample value obtained in step b) with a reference value; and,
   if said sample value is higher than said reference value,
   d) treating said subject with an adjuvant breast cancer treatment regimen.
11. Method according to item 10, wherein said reference value of step c) is associated with a reference prognosis and said breast cancer treatment regimen of step d) is adapted to a prognosis for said subject which is worse than said reference prognosis.
12. Method according to item 10 or 11, wherein said breast cancer treatment regimen is selected from chemotherapy, neo-adjuvant therapy and combinations thereof.
13. Method according to item 12, wherein said treatment regimen is neo-adjuvant therapy.
14. Method according to item 13, wherein said neo-adjuvant therapy is selected from the group consisting of i) radiation therapy only and ii) radiation therapy in combination with chemotherapy.
15. Method according to any one of items 5-9 and 11-14, wherein said prognosis is a probability of having a metastasis, said reference prognosis is a reference probability of having a metastasis and step d) comprises concluding that said probability is higher than said reference probability.
16. Method according to item 15, wherein said metastasis is a distant metastasis.
17. Method according to one of items 5-9 and 11-14, wherein said prognosis is a probability of survival, such as overall survival, recurrence free survival or breast cancer specific survival, and said reference prognosis is a reference probability of survival, such as overall survival, recurrence free survival or breast cancer specific survival, wherein both survivals are the same type of survival.
18. Method according to item 17, wherein said probability of survival is a probability of overall survival and said reference probability of survival is a reference probability of overall survival.
19. Method according to item 17, wherein said probability of survival is a probability of recurrence-free survival and said reference probability of survival is a reference probability of recurrence-free survival.
20. Method according to item 17, wherein said probability of survival is a probability of breast cancer specific survival and said reference probability of survival is a reference probability of breast cancer specific survival.
21. Method according to any one of items 17-20, wherein the probability of survival is a probability of five-year, ten-year or 15-year survival.
22. Method according to any one of the preceding item, wherein said breast cancer is hormone receptor positive, such as ER positive and/or PR positive.
23. Method according to item 22, wherein said breast cancer is ER positive.
24. Method according to any one of the preceding items, wherein said subject has an invasive ductal carcinoma.
25. Method according to any one of the preceding items, wherein said subject is a human.
26. Method according to any one of the preceding items, wherein said subject is a female.
27. Method according to any one of the preceding items, wherein said subject is a premenopausal or postmenopausal human female.
28. Method according to any one of the preceding items, wherein said subject is a premenopausal human female.
29. Method according to any one of the preceding items, wherein said sample is a body fluid sample.
30. Method according to item 29, wherein said body fluid is selected from the group consisting of blood, lymph, plasma, serum, cerebral fluid, urine, semen and exudate.
31. Method according to any one of items 1-28, wherein said sample is a cytology sample.
32. Method according to any one of items 1-28, wherein said sample is a stool sample.
33. Method according to any one of the preceding items, wherein said sample comprises cells from said subject.
34. Method according to any one of the preceding items, wherein said sample comprises tumor cells from said subject.
35. Method according to any one of items 1-28, wherein said sample is a tissue sample.
36. Method according to item 35, wherein said tissue sample is a breast tissue sample.
37. Method according to item 36, wherein said breast tissue sample is a breast cancer tissue sample.
38. Method according to any one of the preceding items, wherein said evaluation of step b) is limited to the nucleus, cytoplasm and/or membrane of cells of said sample.
39. Method according to any one of the preceding items, wherein said evaluation of step b) is limited to the nucleus of cells of said sample.
40. Method according to item 38 or 39, wherein said cells of said sample are tumor cells of said sample.
41. Method according to any one of the preceding items, wherein said reference value is a value corresponding to an amount of ANLN protein in a reference sample.
42. Method according to any one of the preceding items, wherein said sample value of step b) is determined as being either 1, corresponding to detectable ANLN protein in said sample, or 0, corresponding to no detectable ANLN protein in said sample.
43. Method according to any one of the preceding items, wherein said reference value of step c) corresponds to a reference sample having no detectable ANLN protein.
44. Method according to any one of the preceding items, wherein said reference value of step c) is 0.
45. Method according to any one of the preceding items, wherein said reference value is selected from the group consisting of a cytoplasmic fraction, a cytoplasmic intensity, a function of a cytoplasmic fraction and a cytoplasmic intensity, a nuclear fraction, a nuclear intensity and a function of a nuclear fraction and a nuclear intensity.
46. Method according to any one of the preceding items, wherein said reference value is selected from the group consisting of a nuclear fraction, a nuclear intensity and a function of a nuclear fraction and a nuclear intensity.
47. Method according to item 46, wherein said reference value is a nuclear fraction.
48. Method according to item 47, wherein said reference value is a nuclear fraction of 30 % ANLN positive cells, or lower.
49. Method according to item 48, wherein said reference value is a nuclear fraction of 0.5 - 30 % ANLN positive cells.
50. Method according to item 48, wherein said reference value is a nuclear fraction of 15 % ANLN positive cells, or lower.
51. Method according to item 50, wherein said reference value is a nuclear fraction of 10 % ANLN positive cells, or lower.
52. Method according to item 51, wherein said reference value is a nuclear fraction of 1 - 10 % ANLN positive cells.
53. Method according to item 50, wherein said reference value is a nuclear fraction of 1 % ANLN positive cells, or lower.
54. Method according to item 46, wherein said reference value is a nuclear intensity.
55. Method according to item 54, wherein said reference value is a weak nuclear intensity of ANLN protein expression, or lower.
56. Method according to item 55, wherein said reference value is a absent nuclear intensity of ANLN protein expression.
57. Method according to any one of the preceding items, wherein the amino acid sequence of the ANLN protein comprises a sequence selected from:
   i) SEQ ID NO:1; and
   ii) a sequence which is at least 85 % identical to SEQ ID NO:1.
58. Method according to any one of the preceding items, wherein the amino acid sequence of the ANLN protein comprises a sequence selected from:
   i) SEQ ID NO:2; and
   ii) a sequence which is at least 85 % identical to SEQ ID NO:2.
59. Method according to any one of the preceding items, wherein step b) comprises:
   b1) applying to said sample a quantifiable affinity ligand capable of selective interaction with the ANLN protein to be evaluated, said application being performed under conditions that enable binding of said affinity ligand to any ANLN protein present in said sample;
   b2) removing non-bound affinity ligand; and
   b3) quantifying the affinity ligand remaining in association with said sample to evaluate said amount.
60. Method according to item 59, wherein said quantifiable affinity ligand is selected from the group consisting of antibodies, fragments thereof and derivatives thereof.
61. Method according to item 60, wherein said quantifiable affinity ligand is obtainable by a process comprising a step of immunizing an animal with a protein whose amino acid sequence comprises the sequence SEQ ID NO:1.
62. Method according to item 61, wherein said quantifiable affinity ligand is obtainable by a process comprising a step of immunizing an animal with a protein whose amino acid sequence consists of the amino acid sequence SEQ ID NO:1.
63. Method according to item 59, wherein said quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
64. Method according to item 59, wherein said quantifiable affinity ligand is an oligonucleotide molecule.
65. Method according to any one of items 59-64, wherein said quantifiable affinity ligand is capable of selective interaction with an ANLN protein whose amino acid sequence consists of the sequence of SEQ ID NO:1.
66. Method according to any one of items 59-65, wherein said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
67. Method according to any one of items 59-66, wherein said quantifiable affinity ligand is detected using a secondary affinity ligand capable of recognizing said quantifiable affinity ligand.
68. Method according to item 67, wherein said secondary affinity ligand is capable of recognizing said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
69. Method according to any one of items 4-5, which, if said sample value is lower than or equal to said reference value, further comprises a step of:
   e) concluding that said prognosis for said subject is better than or equal to said reference prognosis.
70. Kit for carrying out the method according to any one of the preceding items, which comprises
   a) a quantifiable affinity ligand capable of selective interaction with an ANLN protein; and
   b) reagents necessary for quantifying the amount of said quantifiable affinity ligand.
71. Kit according to item 70, in which said quantifiable affinity ligand is selected from the group consisting of antibodies, fragments thereof and derivatives thereof.
72. Kit according to item 71, in which said quantifiable affinity ligand is obtainable by a process comprising a step of immunizing an animal with a protein whose amino acid sequence comprises the sequence SEQ ID NO:1.
73. Kit according to item 72, in which said quantifiable affinity ligand is obtainable by a process comprising a step of immunizing an animal with a protein whose amino acid sequence consists of the sequence SEQ ID NO:1.
74. Kit according to any of one of items 70, in which said quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
75. Kit according to any of one of items 70, in which said quantifiable affinity ligand is an oligonucleotide molecule.
76. Kit according to any one of items 70-75, in which said quantifiable affinity ligand is capable of selective interaction with an ANLN protein comprising a sequence selected from:
   i) SEQ ID NO:2; and
   a sequence which is at least 85 % identical to SEQ ID NO:2.
77. Kit according to any one of items 70-76, in which said quantifiable affinity ligand is capable of selective interaction with an ANLN protein comprising a sequence selected from:
   i) SEQ ID NO:1; and
   ii) a sequence which is at least 85 % identical to SEQ ID NO:1.
78. Kit according to any one of items 70-76, in which the ANLN protein comprises 200 amino acids or less, such as 150 amino acids or less.
79. Kit according to any one of items 70-78, in which said quantifiable affinity ligand is capable of selective interaction with a polypeptide whose amino acid sequence consists of the sequence SEQ ID NO:1.
80. Kit according to any one of items 70-79, in which said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
81. Kit according to any one of items 70-80, in which said reagents necessary for quantifying said amount of said quantifiable affinity ligand comprise a secondary affinity ligand capable of recognizing said quantifiable affinity ligand.
82. Kit according to item 81, in which said secondary affinity ligand capable of recognizing said quantifiable affinity ligand comprises a label selected from the group consisting of fluorescent dyes or metals, chromophoric dyes, chemiluminescent compounds and bioluminescent proteins, enzymes, radioisotopes, particles and quantum dots.
83. Kit according to any one of items 70-82, further comprising at least one reference sample for provision of a reference value.
84. Kit according to item 83, in which at least one reference sample is a tissue sample comprising no detectable ANLN protein.
85. Kit according to item 83 or 84, in which at least one reference sample comprises ANLN protein.
86. Kit according to any one of items 83-85, in which at least one reference sample comprises an amount of ANLN protein corresponding to a nuclear fraction of 0.5 - 30 %.
87. Kit according to item 86, in which at least one reference sample comprises an amount of ANLN protein corresponding to a nuclear fraction of 1-10%.
88. Kit according to item 83-85, in which at least one reference sample comprises an amount of ANLN protein corresponding to a nuclear fraction of 1 % ANLN positive cells or lower.
89. Kit according to any one of items 83-85, in which at least one reference sample comprises an amount of ANLN protein corresponding to a moderate nuclear intensity of ANLN protein expression or lower.
90. Kit according to item 89, in which at least one reference sample comprises an amount of ANLN protein corresponding to an absent nuclear intensity of ANLN protein expression.
91. Kit according to any one of items 83-90, in which at least one reference sample comprises an amount of ANLN protein corresponding to a value being higher than said reference value.
92. Kit according to item 91, in which at least one reference sample comprises an amount of ANLN protein corresponding to a strong nuclear intensity.
93. Kit according to item 91, in which at least one reference sample comprises an amount of ANLN protein corresponding to a nuclear fraction of 75 % or higher.
94. Kit according to any one of items 83-93 comprising:
   a first reference sample comprising an amount of ANLN protein corresponding to a value (positive reference value) being higher than a reference value; and
   a second reference sample comprising an amount of ANLN protein corresponding to a value (negative reference value) being lower than said reference value.
95. Kit according to any one of items 83-94, in which said reference sample(s) is/are tissue sample(s).
96. Kit according to any one of items 70-95, further comprising
   a') a quantifiable affinity ligand capable of selective interaction with an estrogen receptor; and
   b') reagents necessary for quantifying the amount of the quantifiable affinity ligand of a').
97. Kit according to any one of items 70-96, further comprising
   a") a quantifiable affinity ligand capable of selective interaction with a progesterone receptor; and
   b") reagents necessary for quantifying the amount of the quantifiable affinity ligand of a").
98. ANLN protein consisting of 200 amino acid residues or less, which comprises a sequence selected from:
   i) SEQ ID NO:1; and
   ii) a sequence which is at least 85 % identical to SEQ ID NO:1.
99. ANLN protein according to item 98, which consists of 150 amino acid residues or less.
100. ANLN protein according to item 98 or 99, which comprises a sequence selected from:
   i) SEQ ID NO:1; and
   ii) a sequence which is at least 95 % identical to SEQ ID NO:1.
101. ANLN protein according to item 98, which consists of the amino acid sequence of SEQ ID NO:1.
102. Use of an ANLN protein as an endocrine treatment indicator.
103. Use according to item 102 as an endocrine treatment indicator for a mammalian subject having an ER positive breast cancer.
104. Use of an ANLN protein as a prognostic marker.
105. Use according to item 104 as a prognostic marker for cancer.
106. Use according to item 105, wherein said cancer is a breast cancer.
107. Use according to any one of items 104-106, wherein said prognostic marker is an indicator of the probability of having a breast cancer metastasis, such as a distant metastasis.
108. Use of an ANLN protein, or an antigenically active fragment thereof, for the production, selection or purification of a prognostic agent or an endocrine treatment predictive agent.
109. Use according to item 108, wherein said prognostic agent or endocrine treatment predictive agent is an affinity ligand capable of selective interaction with said ANLN protein.
110. Use according to item 109, wherein said affinity ligand is capable of selective interaction with a protein consisting of the sequence SEQ ID NO:1.
111. Use according to any one of items 108-110, wherein said prognostic agent is for establishing a probability of having a breast cancer metastasis, such as a distant metastasis.
112. Use according any one of items 102-111, wherein the amino acid sequence of the ANLN protein comprises a sequence selected from:
   i) SEQ ID NO:1; and
   ii) a sequence which is at least 85 % identical to SEQ ID NO:1.
113. Use according any one of items 102-112, wherein the amino acid sequence of the ANLN protein comprises a sequence selected from:
   i) SEQ ID NO:2; and
   ii) a sequence which is at least 85 % identical to SEQ ID NO:2.
114. Use according any one of items 102-113, wherein the ANLN protein is the ANLN protein according to any one of items 98-101.
115. Affinity ligand capable of selective interaction with an ANLN protein.
116. Affinity ligand according to item 115, which is obtainable by a process comprising a step of immunizing an animal with a protein whose amino acid sequence comprises the sequence SEQ ID NO:1.
117. Affinity ligand according to item 116, which is obtainable by a process comprising a step of immunizing an animal with a protein whose amino acid sequence consists of the sequence SEQ ID NO:1.
118. Affinity ligand according to any one of items 115-117, capable of selective interaction with an ANLN protein whose amino acid sequence consists of the amino acid sequence SEQ ID NO:1.
119. Affinity ligand according to any one of items 115-118 for use in an *in vivo* method for establishing a prognosis for a mammalian subject having a breast cancer or a prediction of an outcome of an endocrine treatment of a mammalian subject having a breast cancer, such as a ER positive breast cancer.
120. Affinity ligand according to any one of items 115-118, for establishing a treatment prediction for an endocrine treatment of a mammalian subject having a breast cancer, such as a ER+ breast cancer.
121. Affinity ligand according to any one of items 115-118, for establishing a prognosis for a mammalian subject having a breast cancer.
122. Affinity ligand according to item 121, wherein said prognosis is a probability of having a metastasis.
123. Affinity ligand according to any one of items 115-122, for evaluating the amount of ANLN protein present in at least part of a sample from a subject having a breast cancer.
124. Use of an affinity ligand according to any one of items 115-123 as a prognostic agent or a endocrine treatment predictive agent.
125. Use according to item 124, wherein said endocrine treatment predictive agent is for prediction of an outcome of an endocrine treatment of a mammalian subject having a breast cancer, such as a ER positive breast cancer.
126. Use according to item 124, wherein said prognostic agent is for establishing a prognosis for a mammalian subject having a breast cancer.
127. Use according to item 126, wherein said prognosis is a probability of having a metastasis.
128. Use of the affinity ligand according to any one of items 115-123 in the manufacture of a prognostic agent for establishing a prognosis for a mammalian subject having a breast cancer.
129. Use of the affinity ligand according to any one of items 115-123 in the manufacture of a endocrine treatment predictive agent for an endocrine treatment of a mammalian subject having a breast cancer, such as an ER+ breast cancer.
130. Endocrine treatment product for use in the treatment of a mammalian subject having a breast cancer, wherein said subject is hormone receptor positive, such as ER positive and/or PR positive, and ANLN protein negative.
131. Use of an endocrine treatment product in the manufacture of a medicament for treatment of a mammalian subject having a breast cancer, wherein said subject is hormone receptor positive, such as ER positive and/or PR positive, and ANLN protein negative.

### Brief description of the figures

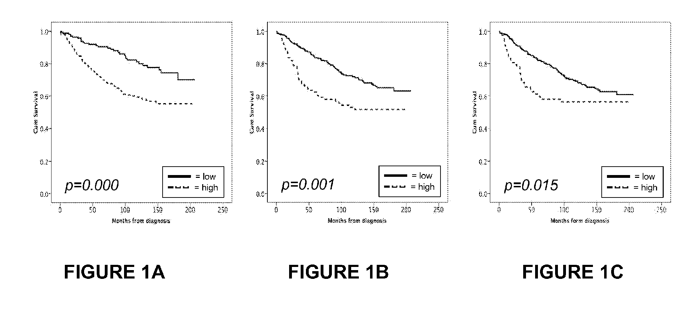

Figure 1 shows the results of a survival analysis based on immunohistochemistry (IHC) staining of 467 subjects diagnosed with breast carcinoma in Cohort I. Tissue cores were scored for high or low ANLN protein level. Figure 1 a shows recurrence free survival (RFS) in all patients, wherein nf > 1 % was considered high and nf ≤ 1 % was considered low. Figure 1b shows RFS in all patients, wherein nf > 10 % was considered high and nf ≤ 10 % was considered low. Figure 1c shows RFS in all patients, wherein nf > 25 % was considered high and nf ≤ 25 % was considered low.

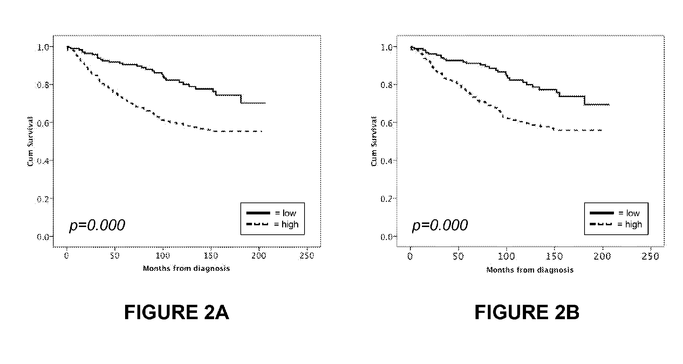

Figure 2 shows the results of a survival analysis based on immunohistochemistry (IHC) staining of 467 subjects diagnosed with breast carcinoma in Cohort I. Tissue cores were scored for high or low ANLN protein level, wherein nf > 1 % was considered high and nf ≤ 1 % was considered low. Figure 2a shows RFS in all 467 patients. Figure 2b shows RFS in 400 patients positive for ER.

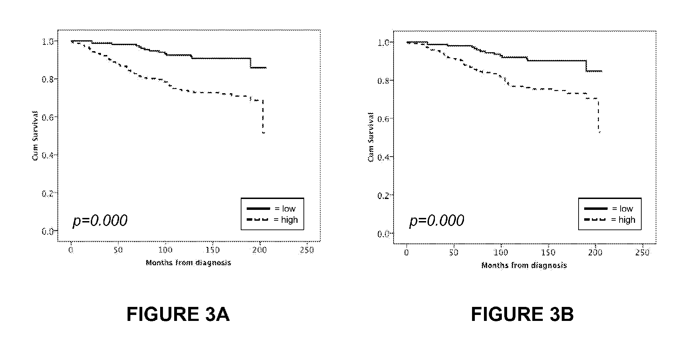

Figure 3 shows the results of a survival analysis based on immunohistochemistry (IHC) staining of 467 subjects diagnosed with breast carcinoma in Cohort I. Tissue cores were scored for high or low ANLN protein level, wherein nf > 1 % was considered high and nf ≤ 1 % was considered low. Figure 3a shows BCSS in all 467 patients. Figure 3b shows BCSS in 400 patients positive for ER.

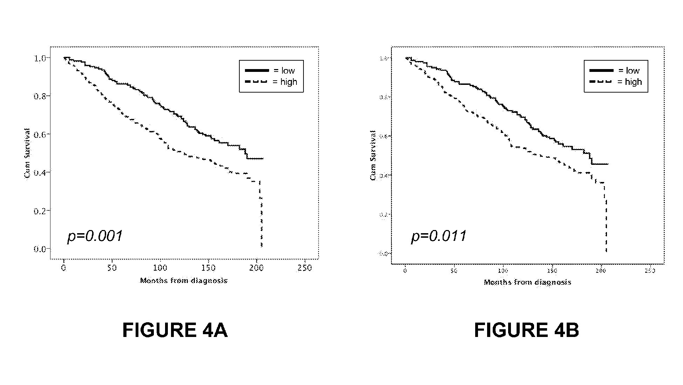

Figure 4 shows the results of a survival analysis based on immunohistochemistry (IHC) staining of 467 subjects diagnosed with breast carcinoma in Cohort I. Tissue cores were scored for high or low ANLN protein level, wherein nf > 1 % was considered high and nf ≤ 1 % was considered low. Figure 4a shows OS in all 467 patients. Figure 4b shows OS in 400 patients positive for ER.

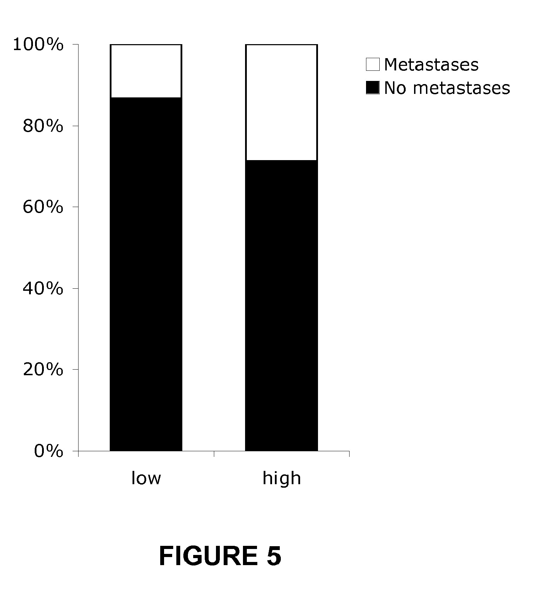

Figure 5 shows shows the frequency of metastases in subjects with low ANLN protein level as compared to subjects with high ANLN protein level, wherein nf > 1 % was considered high and nf ≤ 1 % was considered low.

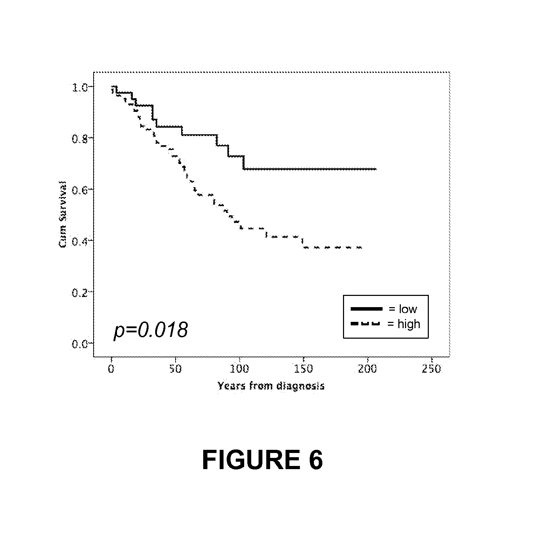

Figure 6 shows RFS based on immunohistochemistry (IHC) staining of 128 ER+ subjects diagnosed with breast carcinoma in Cohort I. All patients were given adjuvant tamoxifen treatment. Tissue cores were scored for high or low ANLN protein level, wherein nf > 1 % was considered high and nf ≤ 1 % was considered low.

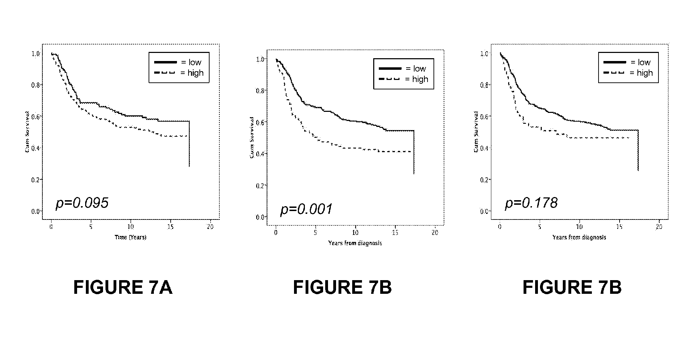

Figure 7 shows the results of a survival analysis based on immunohistochemistry (IHC) staining of 357 subjects diagnosed with breast carcinoma in Cohort II. Tissue cores were scored for high or low ANLN protein level. Figure 7a shows recurrence free survival (RFS) in all patients, wherein nf > 1 % was considered high and nf ≤ 1 % was considered low. Figure 7b shows RFS in all patients, wherein nf > 10 % was considered high and nf ≤ 10 % was considered low. Figure 7c shows RFS in all patients, wherein nf > 25 % was considered high and nf ≤ 25 % was considered low.

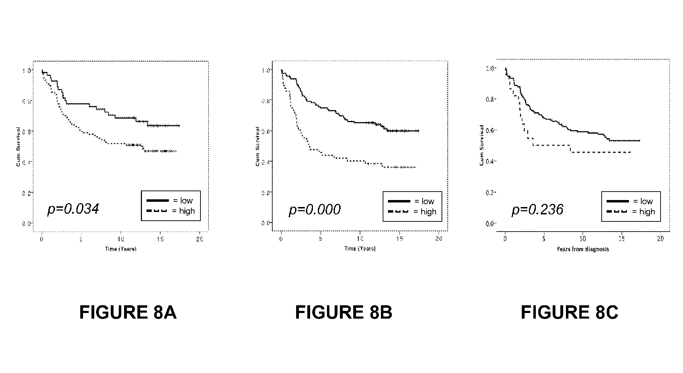

Figure 8 shows the results of a survival analysis based on immunohistochemistry (IHC) staining of 173 subjects diagnosed with breast carcinoma in Cohort II. All patients were receiving adjuvant tamoxifen for 2 years. Tissue cores were scored for high or low ANLN protein level. Figure 8a shows recurrence free survival (RFS) in all patients, wherein nf > 1 % was considered high and nf ≤ 1 % was considered low. Figure 8b shows RFS in all patients, wherein nf > 10 % was considered high and nf ≤ 10 % was considered low.

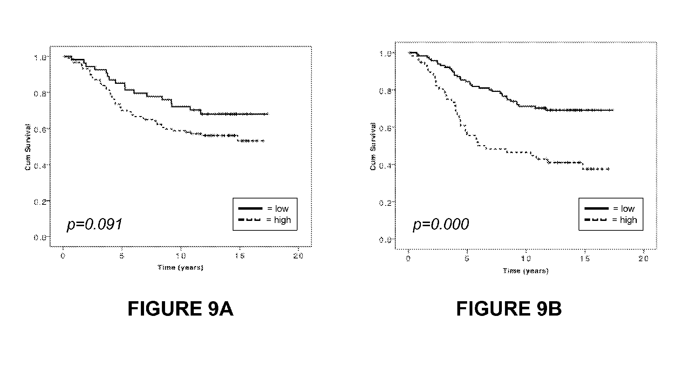

Figure 9 shows the results of a survival analysis based on immunohistochemistry (IHC) staining of 173 subjects diagnosed with breast carcinoma in Cohort II. All patients were receiving adjuvant tamoxifen for 2 years. Tissue cores were scored for high or low ANLN protein level. Figure 9a shows breast cancer specific survival (BCSS) in all patients, wherein nf > 1 % was considered high and nf ≤ 1 % was considered low. Figure 9b shows BCSS in all patients, wherein nf > 10 % was considered high and nf ≤ 10 % was considered low.

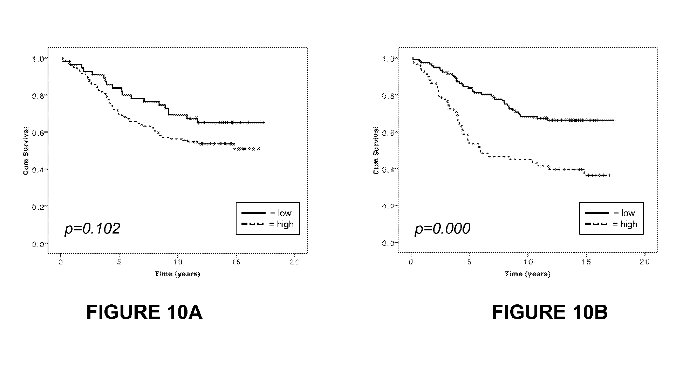

Figure 10 shows the results of a survival analysis based on immunohistochemistry (IHC) staining of 173 subjects diagnosed with breast carcinoma in Cohort II. All patients were receiving adjuvant tamoxifen for 2 years. Tissue cores were scored for high or low ANLN protein level. Figure 10a shows overall survival (OS) in all patients, wherein nf > 1 % was considered high and nf ≤ 1 % was considered low. Figure 10b shows OS in all patients, wherein nf > 10 % was considered high and nf ≤ 10 % was considered low.

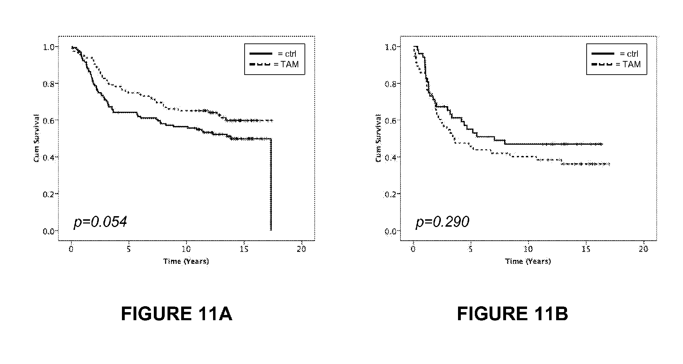

Figure 11 shows the results of a survival analysis based on immunohistochemistry (IHC) staining of 173 subjects diagnosed with breast carcinoma in Cohort II. Tissue cores were scored for high or low ANLN protein level, wherein nf > 10 % was considered high and nf ≤ 10 % was considered low. Further, TAM represents a group of subjects given adjuvant tamoxifen for 2 years, and ctrl represents a control group not given tamoxifen. Figure 11a shows RFS in all patients with low ANLN protein level. Figure 11 b shows RFS in all patients with high ANLN protein level.

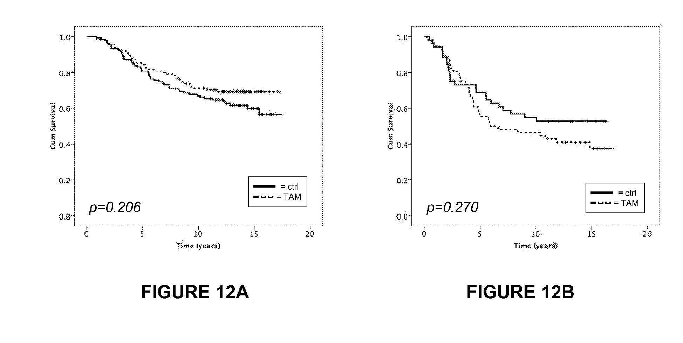

Figure 12 shows the results of a survival analysis based on immunohistochemistry (IHC) staining of 173 subjects diagnosed with breast carcinoma in Cohort II. Tissue cores were scored for high or low ANLN protein level, wherein nf > 10 % was considered high and nf ≤ 10 % was considered low. Further, TAM represents a group of subjects given adjuvant tamoxifen for 2 years, and ctrl represents a control group not given tamoxifen. Figure 12a shows BCSS in all patients with low ANLN protein level. Figure 12b shows BCSS in all patients with high ANLN protein level.

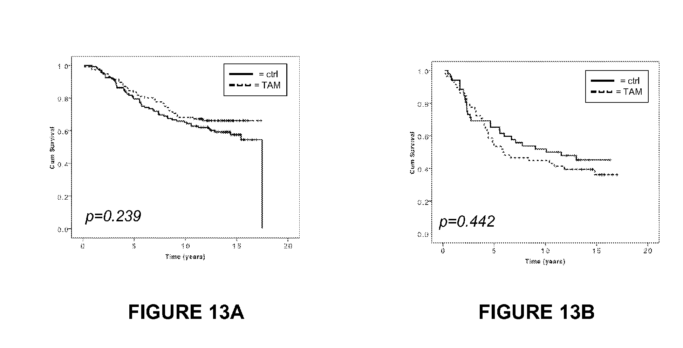

Figure 13 shows the results of a survival analysis based on immunohistochemistry (IHC) staining of 173 subjects diagnosed with breast carcinoma in Cohort II. Tissue cores were scored for high or low ANLN protein level, wherein nf > 10 % was considered high and nf ≤ 10 % was considered low. Further, TAM represents a group of subjects given adjuvant tamoxifen for 2 years, and ctrl represents a control group not given tamoxifen. Figure 13a shows OS in all patients with low ANLN protein level. Figure 13b shows OS in all patients with high ANLN protein level.

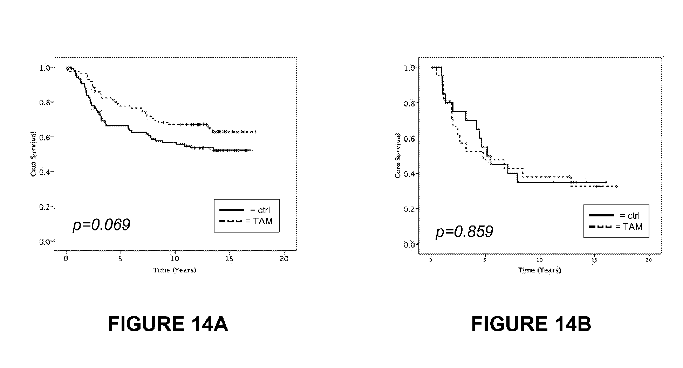

Figure 14 shows the results of a survival analysis based on immunohistochemistry (IHC) staining of 233 ER+ subjects diagnosed with breast carcinoma in Cohort II. Tissue cores were scored for high or low ANLN protein level, wherein nf > 10 % was considered high and nf ≤ 10 % was considered low. Further, TAM represents a group of subjects given adjuvant tamoxifen for 2 years, and ctrl represents a control group not given tamoxifen. Figure 14a shows RFS in 191 patients with low ANLN protein level. Figure 14b shows RFS in 42 patients with high ANLN protein level.

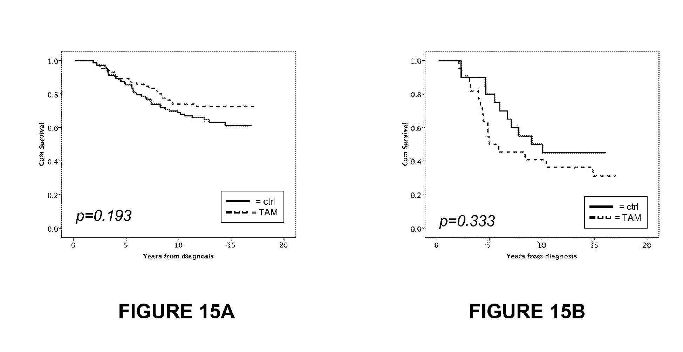

Figure 15 shows the results of a survival analysis based on immunohistochemistry (IHC) staining of 233 ER+ subjects diagnosed with breast carcinoma in Cohort II. Tissue cores were scored for high or low ANLN protein level, wherein nf > 10 % was considered high and nf ≤ 10 % was considered low. Further, TAM represents a group of subjects given adjuvant tamoxifen for 2 years, and ctrl represents a control group not given tamoxifen. Figure 15a shows BCSS in 191 patients with low ANLN protein level. Figure 15b shows BCSS in 42 patients with high ANLN protein level.

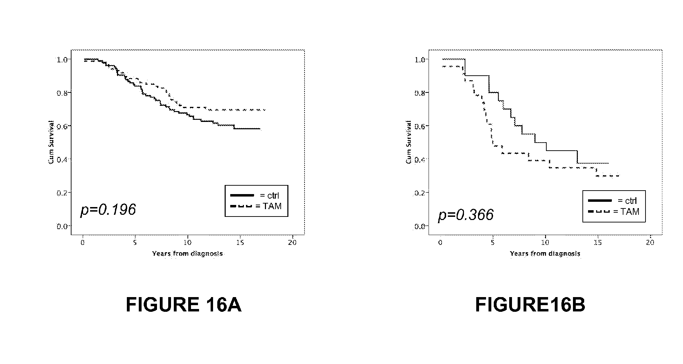

Figure 16 shows the results of a survival analysis based on immunohistochemistry (IHC) staining of 233 ER+ subjects diagnosed with breast carcinoma in Cohort II. Tissue cores were scored for high or low ANLN protein level, wherein nf > 10 % was considered high and nf ≤ 10 % was considered low. Further, TAM represents a group of subjects given adjuvant tamoxifen for 2 years, and ctrl represents a control group not given tamoxifen. Figure 16a shows OS in 191 patients with low ANLN protein level. Figure 16b shows OS in 42 patients with high ANLN protein level.

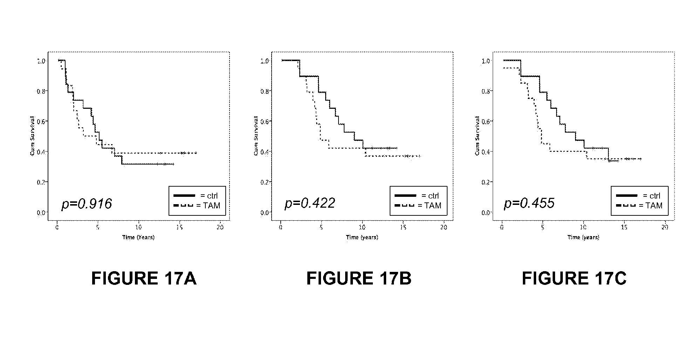

Figure 17 shows the results of survival analyses based on immunohistochemistry (IHC) staining of 202 ER+ subjects diagnosed with invasive ductal carcinoma in Cohort II. Tissue cores were scored for high or low ANLN protein level, wherein nf > 1 % was considered high and nf ≤ 1 % was considered low. Further, TAM represents a group of subjects given adjuvant tamoxifen for 2 years, and ctrl represents a control group not given tamoxifen. Figure 17a shows RFS in 38 patients with high ANLN protein level, figure 17b shows BCSS in 38 patients with high ANLN protein level and figure 17c shows OS in 38 patients with high ANLN protein level.

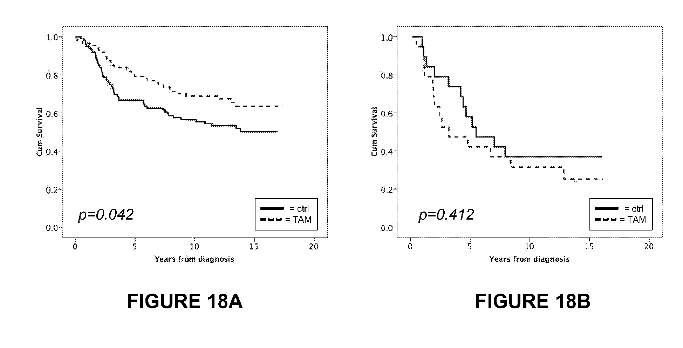

Figure 18 shows the results of a survival analysis based on immunohistochemistry (IHC) staining of 227 PR+ subjects diagnosed with breast carcinoma in Cohort II. Tissue cores were scored for high or low ANLN protein level, wherein nf > 10 % was considered high and nf ≤ 10 % was considered low. Further, TAM represents a group of subjects given adjuvant tamoxifen for 2 years, and ctrl represents a control group not given tamoxifen. Figure 18a shows RFS in 188 patients with low ANLN protein level. Figure 18b shows RFS in 39 patients with high ANLN protein level.

### Detailed description

As a first aspect of the present disclosure, there is provided a method for determining whether a mammalian subject having a breast cancer is likely to benefit from an endocrine treatment, comprising the steps of:
a) providing a sample earlier obtained from the subject;
b) evaluating the amount of ANLN protein present in at least part of the sample, and determining a sample value corresponding to the amount;
c) comparing the sample value obtained in step b) with a reference value; and,
if the sample value is higher than the reference value,
d1) concluding that the subject is not likely to benefit from an endocrine treatment, and/or
   if the sample value is lower than or equal to the reference value,
d2) concluding that the subject is likely to benefit from an endocrine treatment.

In the context of the present disclosure, "likely to benefit from an endocrine treatment" refers to a having a higher probability of survival or recovery if undergoing an endocrine treatment than if not undergoing an endocrine treatment. In this context, "recovery" refers to return from a breast cancer state to a breast cancer free state. The "survival" may be a recurrence free survival or a breast cancer specific survival. Further, the "recovery" may be a recurrence free recovery. Also, the "higher probability" may be a probability benefit at five years, ten years or 15 years of at least 5 %, such as at least 10 %.

However closely related and covered by the same concept, d1) and d2) of the first aspect provide two alternative conclusion options. Accordingly, the method of the first aspect may answer the question whether the subject having a breast cancer is likely to benefit from an endocrine treatment or the question whether the subject having a breast cancer is not likely to benefit from an endocrine treatment. Thus, the method of the first aspect may, but does not have to, comprise both step d1), together with it's adherent qualification ("if phrase"), and step d2), together with it's adherent qualification ("if phrase").

This first aspect of the present disclosure is based on the previously unrecognized fact that the expression of ANLN protein in samples earlier obtained from a subject having a breast cancer may serve as an indicator of response to an endocrine treatment of the subject. More particularly, the present invention identifies, in patients suffering from breast cancer, a correlation between a value of ANLN protein on the one hand and the probability of survival after an endocrine treatment on the other. Typically, high ANLN protein values are shown herein to correlate with a non-responsiveness to endocrine treatment. This aspect, based on ANLN protein expression as an breast cancer treatment indicator, may have a several benefits. Firstly, it may provide a tool for predicting whether a subject is likely to respond to endocrine treatment. Secondly, it may be used to identify a subgroup of breast cancer subjects, that, contrary to earlier beliefs, seems not to respond to endocrine treatment. Consequently, this aspect may provide for accurate treatment of a previously maltreated group. However, this aspect shall not be considered limited to the achievement of the above-mentioned benefits, which are only provided as examples.

As a first alternative configuration of the first aspect of the present disclosure, there is provided a method for determining whether a mammalian subject having a breast cancer should undergo an endocrine treatment, comprising the steps of:
a) providing a sample earlier obtained from the subject;
b) evaluating the amount of ANLN protein present in at least part of the sample, and determining a sample value corresponding to the amount;
c) comparing the sample value obtained in step b) with a reference value; and,
if the sample value is higher than the reference value,
d1) concluding that the subject should not undergo the endocrine treatment, and/or
   if the sample value is lower than or equal to the reference value,
d2) concluding that the subject should undergo the endocrine treatment.

However closely related and covered by the same concept, d1) and d2) of the first configuration of the first aspect provide two alternative conclusion options. Accordingly, the method may answer the question whether the subject should undergo the endocrine treatment or the question whether the subject should not undergo the endocrine treatment. Thus, the method of the first aspect may, but does not have to, comprise both step d1), together with it's adherent qualification ("if phrase"), and step d2), together with it's adherent qualification ("if phrase").

As a second alternative configuration of the first aspect of the present disclosure, there is provided a method for determining whether a sample value corresponding to an amount of ANLN protein present in at least part of a sample earlier obtained from a mammalian subject having a breast cancer is in favor of a decision to give an endocrine treatment to the subject, comprising the steps of:
a) providing a sample earlier obtained from the subject;
b) evaluating the amount of ANLN protein present in at least part of the sample, and determining a sample value corresponding to the amount;
c) comparing the sample value obtained in step b) with a reference value; and,
if the sample value is higher than the reference value,
d1) concluding that the sample value is in favor of a decision to refrain from treating the subject with an endocrine treatment, and/or
   if the sample value is lower than or equal to the reference value,
d2) concluding that the sample value is in favor of a decision to treat the subject with an endocrine treatment.

However closely related and covered by the same concept, d1) and d2) of the second configuration of the first aspect provide two alternative conclusion options. Accordingly, the method may answer the question whether the sample value is in favor of a decision to refrain from treating the subject with an endocrine treatment or the question whether the sample value is in favor of a decision to treat the subject with an endocrine treatment. Thus, the method of the first aspect may, but does not have to, comprise both step d1), together with it's adherent qualification ("if phrase"), and step d2), together with it's adherent qualification ("if phrase").

When deciding on a suitable treatment regimen for a breast cancer patient, the physician responsible for the treatment may take several parameters into account, such as the result of an immunohistochemical evaluation, patient age, menopausal status, hormone receptor status, general condition and medical history, such as breast cancer history. To be guided in such decision, the physician may perform an ANLN protein test, or order an ANLN protein test performed, according to the above configuration.

As a third alternative configuration of the first aspect, there is provided a method for determining whether a prognosis for an endocrine treatment of a mammalian subject having a breast cancer is worse than a reference prognosis, comprising the steps of:
a) providing a sample earlier obtained from the subject;
b) evaluating the amount of ANLN protein present in at least part of the sample, and determining a sample value corresponding to the amount;
c) comparing the sample value obtained in step b) with a reference value; and, if the sample value is higher than the reference value,
d) concluding that the prognosis for endocrine treatment of the subject is worse than the reference prognosis.

Various embodiments of methods for determining whether a prognosis is worse than a reference prognosis are discussed below in connection with the second aspect of the present disclosure.

A physician responsible for the treatment of a subject suffering from breast cancer may, with the knowledge of a prognosis of an endocrine treatment of the subject, decide on whether to give the subject the endocrine treatment or not.

As a second aspect of the present disclosure, there is provided a method of treatment of a subject in need thereof, wherein the subject has a breast cancer, the method comprising the steps of:
a) providing a sample from the subject;
b) evaluating the amount of ANLN protein present in at least part of the sample, and determining a sample value corresponding to the amount;
c) comparing the sample value obtained in step b) with a reference value; and,
if the sample value is higher than the reference value,
d1) treating the subject with a non-endocrine breast cancer treatment regimen, and/or
   if the sample value is lower than or equal to the reference value,
d2) treating the subject with an endocrine treatment regimen.

However closely related and covered by the same concept, d1) and d2) of the second aspect provide two alternative treatment options. Thus, the method of the third aspect may, but does not have to, comprise both step d1), together with it's adherent qualification ("if phrase"), and step d2), together with it's adherent qualification ("if phrase").

Traditionally, endocrine treatment has mainly been given to hormone receptor positive, such as ER positive or PR positive, breast cancer subjects. Consequently, the above methods are especially relevant for this subgroup of subjects. Also the treatment predictive role of ANLN protein in this subgroup is shown in Fig 14-18. Thus, in embodiments of the first or second aspects of the present disclosure, the breast cancer may be a hormone receptor positive breast cancer, such as ER positive and/or PR positive breast cancer.

Within the art, ER status have often been considered the most important indicator of responsiveness to endocrine treatment, such as to tamoxifen treatment. The treatment predictive role of ANLN protein in the subgroup of ER positive subjects is shown in Fig 14-17. Thus, in embodiments of the first or second aspects of the present disclosure, the breast cancer may be ER positive.

If not otherwise stated, "ER" refers to "ERα" in the context of the present disclosure.

The person skilled in the art knows how to obtain the ER and/or PR status of a patient. For example, such information may be obtained from the result of a test using the commercially available ER/PR pharmDX kit (DakoCytomation). As another example, the method disclosed by Allred *et al*. (Allred *et al*. (1998) Mod Pathol 11(2), 155) may be used to obtain a total score (Allred score), and, for both ER and PR, an Allred score of higher than two is considered positive and an Allred score of two or lower is considered negative. Alternatively, when classifying a sample as being positive or negative for ER or PR, a cutoff of 10% positive cells may be used, which is a recognized limit within the art.

For example, the non-endocrine treatment of the second aspect of the present disclosure may be chemotherapy, radiation therapy or a combination thereof. The chemotherapy may be mono- or polychemotherapy, such as treatment with CMF (cyclophosphamide, methotrexate, 5-fluorouracil), FEC (fluorouracil, epirubicin, cyclofosfamid) and/or taxanes. Also, if the breast cancer is HER2 positive (HER2+), the non-endocrine treatment may be an anti-HER2 treatment, such as a treatment with an anti-HER2 antibody, e.g., trastuzumab.

As a third aspect of the present disclosure, there is provided a method for determining whether a prognosis for a mammalian subject having a breast cancer is worse than a reference prognosis, comprising the steps of:
a) providing a sample earlier obtained from the subject;
b) evaluating the amount of ANLN protein present in at least part of the sample, and determining a sample value corresponding to the evaluated amount;
c) comparing the sample value obtained in step b) with a reference value associated with the reference prognosis; and,
d) if the sample value is higher than the reference value, concluding that the prognosis for the subject is worse than the reference prognosis.

In an embodiment, the method of third aspect may comprise the additional step:
e) if the sample value is lower than or equal to the reference value, concluding that the prognosis for the subject is better than or equal to the reference prognosis.

This third aspect of the present disclosure is based on the previously unrecognized fact that the amount of ANLN protein present in samples earlier obtained from a subject having a breast cancer may serve as a disease status indicator in the subject. More particularly, in subjects suffering from a breast cancer, a correlation between an amount of ANLN protein on the one hand and a prognosis for survival on the other are realized by the inventors. Typically, high ANLN protein values are shown herein to correlate with a poor prognosis in these subjects. The present invention based on ANLN protein expression as a cancer status indicator has a number of benefits. In general, early identification of the aggressiveness of a breast cancer is of vital importance as it helps a physician selecting an appropriate treatment strategy. For example, by identifying less aggressive forms at an early stage, over-treatment may be avoided. As a further example, the ANLN protein, as a marker for which a certain level of expression is correlated with a certain pattern of disease progression, may have a great potential for example in a panel for differential diagnostics of a primary tumor. However, this aspect shall not be considered limited to the achievement of the above-mentioned benefits, which are only provided as examples.

Thus, this third aspect generally relates to the same concept as the first: a correlation between ANLN protein levels and a prediction of an outcome of a breast cancer disease. Further, both the third and the first aspect comprise the same practical steps, i.e. the provision of the sample, the evaluation of the ANLN protein amount and determination of the sample value and, finally, the comparison with the reference value.

In the present disclosure, different ANLN protein values (sample values) corresponding to various prognoses are presented. Typically, a high sample value is associated with a poorer prognosis than a low sample value. In the methods according to the third aspect, the sample value is compared to a reference value, and if the sample value is higher than the reference value, it is concluded that the prognosis for the subject is worse than a reference prognosis associated with the reference value.

Consequently, the methods of the above aspect may be adapted to a given reference value. In such case, starting from a given sample value which under certain circumstances is considered to be relevant, a reference value which is equal to, or lower than, that sample value, may be selected. Subsequently, a reference prognosis being associated with that reference value may be established. Guided by the present disclosure, the person skilled in the art should understand how to establish a reference prognosis which corresponds to a given reference value. For example, the relationship between sample values and survival data in a group of breast cancer patients may be examined as in Examples, section 4, below, and the procedure described therein may be adapted to a given reference value, and a prognosis corresponding to the given reference value may then be selected as the reference prognosis.

Also, the methods of the above aspect may be adapted to a given reference prognosis. In such case, starting from a given reference prognosis which under certain circumstances is considered to be relevant, for example for selecting an appropriate treatment strategy, a corresponding reference value may be established. Guided by the present disclosure, the person skilled in the art should understand how to establish a reference value which corresponds to a given reference prognosis. For example, the relationship between sample values and survival data in a group of cancer patients may be examined as in Examples, section 4, below, but the procedure described therein is adapted to establish reference values corresponding to a given reference prognosis. For example, different reference values may be tested until one which correlates with the given reference prognosis is found. A skilled person may do such testing without undue burden, e.g., because the present disclosure presents suitable starting values which may reduce the required amount of testing.

Accordingly, in embodiments of the methods of the above aspect, the reference prognosis may be based on a previously established prognosis, e.g., obtained by an examination of the same subject or population of subjects. Further, the reference prognosis may be adapted to a background risk in the general population, a statistical prognosis/risk or an assumption based on an examination of the subject. Such examination may comprise the subject's age, general condition, sex, race and/or medical status and history, such as cancer history or breast cancer status. For example, a physician may adapt the reference prognosis to the subject's breast cancer history, the stage of the tumor, the hormone receptor status, the morphology of the tumor, the location of the tumor, the presence and spread of metastases and/or further cancer characteristics.

Examples of hormone receptor status are ER status and PR status. For example, ER positivity is generally associated with a relatively good prognosis within the art. As may be seen in e.g., Fig 2b, 3b, 4b and 6, the level of ANLN protein expression provides prognosticly relevant subgroups of ER positive subjects.

Thus, in embodiments of the third aspect, the breast cancer may be hormone receptor positive, such as ER+ and/or PR+.

As shown in Fig 6 and 8-10, the prognostic role of ANLN protein is particularly accentuated for subjects treated with tamoxifen, which is an endocrine treatment. Thus, in embodiments of the third aspect, the subject may have undergone, be undergoing and/or be scheduled for endocrine treatment. As explained above, most subjects undergoing endocrine treatment have hormone receptor positive breast cancers.

In the context of the present disclosure, an "endocrine treatment" refers to a systemic treatment having an anti-estrogenic effect. Further, "anti-estrogenic effect" refers to suppression of estrogen production or inhibition of estrogen effects in the body. In the art, an endocrine treatment is sometimes referred to as an anti-hormonal treatment.

In embodiments of the methods of the above aspects, the endocrine treatment may be selected from the group consisting of a selective estrogen receptor modulator (SERM) treatment, an aromatase inhibitor treatment, and a steroidal estrogen receptor antagonist treatment. The SERM treatment may be a treatment with e.g. toremifene, raloxifene, droloxifene, arzoxifene or tamoxifen. The aromatase inhibitor treatment may be a treatment with e.g. anastrozole, letrozole and exemestane. Further, the steroidal estrogen receptor antagonist treatment may be a treatment with fulvestrant.

A SERM may have either an agonistic or antagonistic effect depending on the target tissue. For example, a SERM may act as an antagonist in breast and as an agonist in uterus.

In embodiments of the methods of the above aspects, the endocrine treatment may be a SERM treatment, such as a SERM treatment selected from treatments with toremifene, raloxifene, droloxifene, arzoxifene or tamoxifen. For example, the endocrine treatment may be tamoxifen treatment.

As a first alternative configuration of the third aspect of the present disclosure, there is provided a method for revising a preliminary prognosis for a mammalian subject having a breast cancer, comprising the steps of:
a) providing a sample earlier obtained from the subject;
b) evaluating the amount of ANLN protein present in at least part of the sample, and determining a sample value corresponding to the evaluated amount;
c) comparing the sample value obtained in step b) with a reference value; and,
   if the sample value is higher than the reference value,
d) lowering the preliminary prognosis to obtain a revised prognosis.

As a second alternative configuration of the third aspect, there is provided a method for establishing a prognosis for a mammalian subject having a breast cancer:
a) providing a sample from the subject;
b) evaluating the amount of ANLN protein present in at least part of the sample, and determining a sample value corresponding to the evaluated amount;
c) correlating the sample value of step b) to the prognosis for the subject.

In the context of the present disclosure, "establishing a prognosis " refers to establishing a specific prognosis or a prognosis interval.

In an embodiment of the above method, the sample may be an earlier obtained sample.

The correlating of step c) refers to any way of associating survival data to the obtained sample value so as to establish a prognosis for the subject.

The identified correlation between ANLN protein levels and breast cancer characteristics may also form the basis for applying various treatment regimes.

As fourth aspect of the present disclosure, there is provided a method of treatment of a subject in need thereof, wherein the subject is having a breast cancer, comprising:
a) providing a sample from the subject;
b) evaluating the amount of ANLN protein present in at least part of the sample, and determining a sample value corresponding to the evaluated amount;
c) comparing the sample value obtained in step b) with a reference value; and
d) if the sample value is higher than the reference value, treating the subject with an adjuvant breast cancer treatment regimen.
   In one embodiment of the fourth aspect, the method may comprise the additional step:
   e) if the sample value is lower than or equal to the reference value, refraining from treating the subject with the adjuvant breast cancer treatment regimen.

In embodiments of the fourth aspect, the reference value of step c) may be associated with a reference prognosis and the breast cancer treatment regimen of step d) may be adapted to a prognosis for the subject which is worse than the reference prognosis. In such an embodiment, the method may comprise the additional step: e) and if the sample value is lower than or equal to the reference value, treating the subject with a treatment regimen adapted to a prognosis which is better than or equal the reference prognosis.

Preferably, the breast cancer treatment regimen of the fourth aspect comprises a non-endocrine treatment. For example, it may be adapted to an aggressive form of breast cancer. The treatment adapted to an aggressive form of cancer may for example be a treatment which would not have been considered if the sample value was not higher than the reference value because of side-effects of such treatment.

In embodiments of the fourth aspect, the breast cancer treatment regimen of step d) may be selected from chemotherapy, neo-adjuvant therapy and combinations thereof. For example, the neo-adjuvant therapy may comprise radiation therapy.

In embodiments of the fourth aspect, the breast cancer may be hormone receptor positive, such as ER positive and/or PR positive. As explained above, hormone receptor positive subjects have traditionally been given endocrine treatment, such as tamoxifen. However, in the light of the present disclosure, it appears more reasonable to give such subjects an alternative, or at least complementary, treatment. Thus, in embodiments of the fourth aspect, the adjuvant breast cancer treatment regimen of step d) in not solely SERM treatment, such as tamoxifen.

Also, in the light of the present disclosure, it may be reasonable to avoid endocrine treatment of some subjects.

Thus, as fifth aspect of the present disclosure, there is provided a non-treatment strategy method for a subject having a breast cancer, comprising the steps of:
a) providing a sample earlier obtained from the subject;
b) evaluating the amount of ANLN protein present in at least part of the sample, and determining a sample value corresponding to the evaluated amount;
c) comparing the sample value obtained in step b) with a reference value; and,
   if the sample value is higher than the reference value,
d) refraining from treating the subject with an endocrine treatment.

For reasons evident from the above, the endocrine treatment of step d) may be a SERM treatment, such as tamoxifen treatment. Also, the breast cancer of the fifth aspect may be hormone receptor positive, such as ER positive and/or PR positive.

For example, the refraining of step d) may be a refraining of at least one week from the completion of steps a) - c), such as at least one month from the completion of steps a) - c), such as at least three months from the completion of steps a) - c), such as at least six months from the completion of steps a) - c), such as at least one year from the completion of steps a) - c), such as at least two years from the completion of steps a) - c).

Alternatively, the refraining of step d) may be a refraining until the next time the method is performed or until recurrence of a breast cancer tumor.

In general, when deciding on a suitable treatment strategy for a patient having breast cancer, the physician responsible for the treatment may take several parameters into account, such as the result of an immunohistochemical evaluation, patient age, hormone receptor status, general condition, medical history, such as breast cancer history and hereditary characteristics, e.g. whether there is a history of breast cancer in the subject's family. To be guided in such decision, the physician may perform ANLN protein test, or order an ANLN protein test performed, according to an embodiment of one of the method aspects presented above.

In the context of the present disclosure, "a mammalian subject having a breast cancer" refers to a mammalian subject having a primary or secondary breast tumor or a mammalian subject which has had a tumor removed from the breast, wherein the removal of the tumor refers to killing or removing the tumor by any type of surgery or therapy. In the latter case, the tumor may for example have been removed less than one year ago. For example, a subject who has had a breast tumor removed by surgery and is about to get adjuvant therapy is considered as "having a breast cancer" in the context of the present disclosure. "Breast tumor" includes ductal carcinoma in situ (DCIS). In the method and use aspects of the present disclosure, "a mammalian subject having a breast cancer" also includes the case wherein the mammalian subject is suspected of having a breast cancer at the time of the performance of the use or method and the breast cancer diagnosis is established later.

In the context of the method aspects of the present disclosure, "earlier obtained" refers to obtained before the method is performed. Consequently, if a sample earlier obtained from a subject is provided in a method, the method does not involve obtaining the sample from the subject, i.e., the sample was previously obtained from the subject in a step separate from the method.

Accordingly, all methods and uses of the present disclosure, may be performed entirely *in vitro.*

Step b) of the methods of the above aspects involve evaluating the amount of ANLN protein present in at least part of the sample, and determining a sample value corresponding to the amount. The "at least part of the sample" refers to a relevant part, or relevant parts, of the sample for establishing the prognosis or drawing conclusions regarding suitable treatments. The person skilled in the art understands which part or parts that are relevant under the circumstances present when performing the method. For example, if the sample comprises tumor and non-tumor cells, the skilled person may only consider the tumor cells, and only the nuclei of the tumor cells, of the sample.

Further, in step b) an amount is evaluated and a sample value corresponding to the amount is determined. Consequently, an exact measurement of the amount of ANLN protein is not required for obtaining the sample value. For example, the amount of ANLN protein may be evaluated by visual inspection of a stained tissue sample and the sample value may then be categorized, e.g., as high or low based on the evaluated amount. The person skilled in the art understands how to perform such evaluation and determination.

For example, the evaluation and determination may be performed as described below in connection with "quantification of ANLN protein expression in a sample".

Regarding step b) of the methods according to the above aspects, an increase in the amount of ANLN protein typically results in an increase in the sample value. However, in some embodiments, the evaluated amount may correspond to any of a predetermined number of discrete sample values. In such embodiments, an first amount and a second, increased, amount, may correspond to the same sample value. In any case, an increase in the amount of ANLN protein will not result in a decrease in the sample value in the context of the present disclosure.

However inconvenient, but in an equivalent fashion, the evaluated amounts may be inversely related to sample values if the qualification between step c) and d) is "if the sample value is lower than the reference value".

Still further, in the context of the present disclosure, the "reference value" refers to a predetermined value which is relevant for making decisions, or drawing conclusions, regarding the prognosis, or making treatment predictions, for the subject.

Also, in the context of the present disclosure, a reference prognosis being "associated" with a reference value refers to the reference value being assigned to the reference prognosis, based on empirical data and/or clinically relevant assumptions. See e.g., step c) of the methods of the third, and certain embodiments of the fourth, aspect. The reference value does not have to be assigned to a reference prognosis directly derived from prognosis data of a group of subjects exhibiting the reference value. The reference prognosis may for example correspond to the prognosis for subjects exhibiting the reference value or lower. That is, if the reference value is 1 on a scale from 0 to 3, the reference prognosis may be the prognosis of the subjects exhibiting the values 0 or 1. Consequently, the reference prognosis may also be adapted to the nature of the available data. As further discussed above, the reference prognosis may be adapted to other parameters as well.

In embodiments of the methods of the above aspects, the subject may have an invasive ductal carcinoma (se also Fig 17).

The data of the present disclosure is based on groups of human females. Thus, in embodiments of the methods of the above aspects, the subject may be a human. Also, in embodiments of the methods of the above aspects, the subject may be a female, such as a premenopausal or postmenopausal female. For example, endocrine treatment has been given to both premenopausal and postmenopausal subjects. In some embodiments, in particular those wherein tamoxifen is selected as the endocrine treatment, premenopausal human female subjects may be a particularly relevant group.

In embodiments of the methods of the above aspects, the sample may be a body fluid sample, such as a sample of blood, plasma, serum, cerebral fluid, lymph, urine or exudate. Preferably, the body fluid sample is sample of blood, plasma, serum or lymph. Alternatively, the sample may be a cytology sample or a stool sample. The body fluid, cytology or stool sample may for example comprise cells, such as tumor cells.

In further embodiments of the methods of the above aspects, the sample may be a tissue sample, such as a tumor tissue sample. As an example, the tissue sample may be derived from a primary breast tumor, such as an in situ or invasive carcinoma, or a secondary tumor (metastasis) from the subject.

ANLN protein expression has been observed in the membrane, cytoplasm and nucleus of cells. Thus, in embodiments of the methods of the above aspects, the evaluation of step b) may be limited to the nucleus, cytoplasm and/or membrane of cells, such as tumor cells, of the sample. When the evaluation is limited to the nucleus, only the characteristics, such as the ANLN protein expression, of the nucleus is considered in the evaluation. Such evaluation may for example by aided by immunohistochemical staining. The strongest staining has been observed in the nucleus. Further, the inventors have found that the nuclear expression of ANLN protein may be relevant for treatment prediction and prognostics. Consequently, in embodiments of the methods of the above aspects, the evaluation of step b) may be limited to the nucleus, of cells, such as tumor cells, of the sample.

As shown in Fig 5, metastases were shown to be more abundant in a group of ANLN protein high subjects than in a group of ANLN protein low subjects.

Thus, in embodiments of the method of the third aspect the prognosis may be a probability of having a metastasis, the reference prognosis may be a reference probability of having a metastasis and step d) may comprise concluding that the probability is higher than the reference probability.

Also, in alternative configurations of such embodiments, the prognosis may be a probability of developing a metastasis, the reference prognosis may be a reference probability of developing a metastasis and step d) may comprise concluding that the probability is higher than the reference probability.

For example, the metastasis may be a distant metastasis.

The conclusion that the probability of having or developing a metastasis may be followed by an metastasis examination aiming at finding possible metastases. Such metastasis examamination may for example be scintigraphy (of the skeleton), X-ray examination of a lung and/or CT scanning.

Consequently, there is provided a method for determining whether a breast cancer in a subject is metastazing, comprising the steps of:
a) providing a sample earlier obtained from the subject;
b) evaluating the amount of ANLN protein present in at least part of the sample, and determining a sample value corresponding to the evaluated amount;
c) comparing the sample value obtained in step b) with a reference value; and,
   if the sample value is higher than the reference value,
d) perform a metastasis examination to find possible metastases is said subject.

In the context of the present disclosure, "prognosis" refers to the prediction of the course or outcome of a disease and its treatment or to the survival of the subject suffering from the disease. For example, prognosis may refer to a determination of chance of survival or recovery from a disease, as well as to a prediction of the expected survival time of a subject. It may also be the likelihood of disease recurrence, e.g., local, regional or distant events. Further, a prognosis may involve establishing the likelihood for survival of a subject during a period of time into the future, such as three years, five years, ten years, fifteen years or any other period of time. Consequently, the prognosis for a subject may for example be a probability of survival, and there are several recognized survival measures. Three different "survival measures" which are commonly used in connection with breast cancer are discussed above, i.e. recurrence free survival, breast cancer specific survival and overall survival.

In the aspects above involving a prognosis and a reference prognosis, these two prognoses are the same type of prognosis. As an example, if the reference prognosis is a reference probability of over-all survival, it follows that the conclusion will be a probability of over-all survival for the subject, which will be in relation to the reference probability over-all survival.

Thus, in embodiments of the methods of the third and fourth aspect, the prognosis may be a probability of survival, which entails that the prognosis for the subject is a probability of survival and the reference prognosis is a probability of survival. For example, the probability of survival may be a probability of five-year survival, ten-year survival or 15-year survival. Further, the probability of survival may for example be selected from the group consisting of probability of overall survival, probability of recurrence-free survival and probability of breast cancer specific survival.

As indicated in the attached figures, the correlation between high ANLN protein levels and a poor prognosis may be particularly accentuated if recurrence-free survival (RFS) is analyzed. Consequently, in embodiments of the methods of the above aspects, the prognosis may be a probability of recurrence-free survival.

A sample value of ANLN protein being higher than the reference value, or a subject from which such sample value is obtained, is sometimes referred to herein as "ANLN protein high". Further, a sample value of ANLN protein being lower than, or equal to, the reference value, or a subject from which such sample value is obtained, is sometimes referred to herein as "ANLN protein low".

In the context of the present disclosure, the terms "sample value" and "reference value" are to be interpreted broadly. The quantification of ANLN protein to obtain these values may be done via automatic means, via a scoring system based on visual or microscopic inspection of samples, or via combinations thereof. However, it is also possible for a skilled person, such as a person skilled in the art of histopathology, to determine the sample and reference values merely by inspection, e.g., of tissue slides that have been stained for ANLN protein expression. The determination of the sample value being higher than the reference value may thus correspond to the determination, upon visual or microscopic inspection, that a sample tissue slide is more densely stained and/or exhibit a larger fraction of stained cells than is the case for a reference tissue slide. The sample value may also be compared to a reference value given by a literal reference, such as a reference value described in wording. Consequently, the sample and/or reference values may be thought of as mental values that the skilled person determines upon inspection and comparison.

For example, the skilled person may categorize a sample as being ANLN protein high or low, wherein the sample is categorized as high if it contains more ANLN protein than a previously inspected reference sample and low if it contains less or equally much. Such evaluation may be assisted by staining the sample, and, if necessary, a reference sample, with a staining solution comprising e.g., antibodies selective for ANLN protein.

A reference value found to be relevant for the provision of a prognosis for a subject having a breast cancer, or for making treatment decisions regarding such subjects, for use as comparison with the sample value from the subject, may be provided in various ways. With the knowledge of the teachings of the present disclosure, the skilled artisan can, without undue burden, provide relevant reference values for performing the methods of the above aspects.

The person performing the methods of the above aspects may, for example, adapt the reference value to desired prognostic information. For example, the reference value may be adapted to yield the most significant prognostic information, e.g., the largest separation between the ANLN protein high survival curve and the ANLN protein low survival curve (see e.g., Fig 1 or 8). Examples of reference values that may yield a large separation are a nuclear fraction of 1 % or 10 %.

Alternatively, the reference value may be adapted to identify a group of subjects having a predetermined prognosis, e.g., the group of subjects having a probability of five-year recurrence-free survival of lower than 85 %.

In embodiments of the methods of the above aspects, the reference value may correspond to the amount of ANLN protein expression in a healthy tissue, such as healthy breast or stroma tissue, of the subject of the method. As another example, the reference value may be provided by the amount of ANLN protein expression measured in a standard sample of normal tissue from another, comparable subject. As another example, the reference value may be provided by the amount of ANLN protein expression measured in a reference sample comprising tumor cells, such as a reference sample of tumor tissue, e.g., breast cancer tissue. The amount of protein expression of the reference sample may preferably be previously established. Consequently, the reference value may be provided by the amount of ANLN protein measured in a reference sample comprising cells expressing a predetermined amount of ANLN protein.

Further, the reference value may for example be provided by the amount of ANLN protein expression measured in a reference sample comprising cell lines, such as cancer cell lines, expressing a predetermined, or controlled, amount of ANLN protein. The person skilled in the art understands how to provide such cell lines, for example guided by the disclosure of Rhodes et al. (2006) The biomedical scientist, p 515-520.

Consequently, in embodiments of the methods of the above aspects, the reference value may be a predetermined value corresponding to the amount of ANLN protein expression in a reference sample.

However, as discussed further below, the amount of ANLN protein in the reference sample does not have to directly correspond to the reference value. The reference sample may also provide amounts of ANLN protein that help a person performing the method to assess various reference values. For example, the reference sample(s) may help in creating a mental image of the reference value by providing a "positive" (high) and/or a "negative" (low) value.

The inventors show herein that subjects who suffer from breast cancer and have essentially no ANLN protein expression generally have a relatively good prognosis. Thus, in embodiments of the methods of the above aspects, the sample value of step b) may be either 1, corresponding to detectable ANLN protein in the sample, or 0, corresponding to no detectable ANLN protein in the sample. Consequently, in such embodiments, the evaluation of the sample is digital: ANLN protein is considered to be either present or not. In the context of the present disclosure, "no detectable ANLN protein" refers to an amount of ANLN protein that is so small that it is not, during normal operational circumstances, detectable by a person or an apparatus performing the method according to any one of the above aspects. The "normal operational circumstances" refer to the laboratory methods and techniques a person skilled in the art would find appropriate for performing the invention.

Accordingly, in embodiments of the methods of the above aspects, the reference value of step c) may be 0. And it follows that, in further embodiments of the methods of the above aspects, the reference value of step c) may correspond to a reference sample having no detectable ANLN protein. This means that the reference value may be obtained by analysis of a reference sample lacking ANLN protein.

One alternative for the quantification of ANLN protein expression in a sample, such as the sample earlier obtained from the subject or the reference sample, is the determination of the fraction of cells in the sample that exhibit ANLN protein expression over a certain level. The fraction may for example be: a "cellular fraction", wherein the ANLN protein expression of the whole cells is taken into account; a "cytoplasmic fraction", wherein the ANLN protein expression of only the cytoplasms of the cells is taken into account; or a "nuclear fraction", wherein the ANLN protein expression of only the nuclei of the cells is taken into account. The nuclear fraction may for example be classified as ≤ 1 %, 2 - 25 %, 26 - 75 % or > 75 % immunoreactive cells of the relevant cell population. Alternatively, the nuclear fraction may be classified as ≤ 10 % or > 10 - 100 %, or as ≤ 1 % or > 1 - 100 %. This determination may for example be performed as described below in the Examples, Section 4, with reference to "nuclear fraction". The "nuclear fraction" corresponds to the percentage of relevant cells in a sample that exhibits a positive staining in the nucleus, wherein a medium or distinct and strong immunoreactivity in the nucleus is considered positive and no or faint immunoreactivity in the nucleus is considered negative. The person skilled in the art of pathology understands which cells that are relevant under the conditions present when performing the method and may determine a nuclear fraction based on his general knowledge and the teachings of the present disclosure. The relevant cells may for example be tumor cells. Further, the skilled artisan understands how to perform corresponding measurements employing the "cellular fraction" or the "cytoplasmic fraction".

Another alternative for the quantification of ANLN protein expression in a sample, such as the sample earlier obtained from the subject or the reference sample, is the determination of the overall staining intensity of the sample. The intensity may for example be: a "cellular intensity", wherein the ANLN protein expression of the whole cells is taken into account; a "cytoplasmic intensity", wherein the ANLN protein expression of only the cytoplasms of the cells is taken into account, or a "nuclear intensity", wherein the ANLN protein expression of only the nuclei of the cells is taken into account. Nuclear intensity is subjectively evaluated in accordance with standards used in clinical histopathological diagnostics. Outcome of a nuclear intensity determination may be classified as: absent = no overall immunoreactivity in the nucleus of relevant cells of the sample, weak = faint overall immunoreactivity in the nucleus of relevant cells of the sample, moderate = medium overall immunoreactivity in the nucleus of relevant cells of the sample, or strong = distinct and strong overall immunoreactivity in the nucleus of relevant cells of the sample. The person skilled in the art understands which cells that are relevant under the conditions present when performing the method and may determine a nuclear intensity based on his general knowledge and the teachings of the present disclosure. The relevant cells may for example be tumor cells. Further, the skilled artisan understands how to perform corresponding measurements employing the "cellular intensity" or the "cytoplasmic intensity".

The inventors have found that the nuclear expression of ANLN protein is particularly relevant for establishing a prognosis. Thus, in embodiments of the methods of the above aspects, the reference value may be a nuclear fraction, a nuclear intensity or a combination thereof. Accordingly, the sample value may be a nuclear fraction, a nuclear intensity or a combination thereof.

Preferably, the sample value and the reference value are both the same type of value. Accordingly, in embodiments of the methods of the above aspects, the sample value and the reference value may each be a nuclear fraction, a nuclear intensity or a combinations thereof.

In embodiments of the methods of the above aspects, the criterion for the conclusion in step d) may be a sample value for the nuclear fraction of ANLN protein positive cells, i.e., a "nuclear fraction", which is higher than a reference value which is 0.5 %, such as higher than 1 %, such as higher than 2 %, such as higher than 5 %, such as higher than 10 %, such as higher than 15 %, such as higher than 20 %, such as higher than 25 %, such as higher than 30 %, such as higher than 35 %, such as higher than 40 %, such as higher than 50 %, such as higher than 55 %, such as higher than 60 %, such as higher than 65 %, such as higher than 70 %, such as higher than 75 %, such as higher than 80 %, such as higher than 85 %, such as higher than 90 %, such as higher than 95 %.

In alternative or complementing embodiments of the methods of the above aspects, the reference value of step c) is a nuclear fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower.

As shown in the attached figures, in particular Fig 1 and 8, cut-off:s of nf 1 %, 10 % or 25 %, provide relevant prognostic or treatment predictive information. A nf of 10 % appears to be a particularly promising cut-off.

Thus, in preferred embodiments, the reference value of step c) may be a nuclear fraction of 35 % or lower, such as 30 % or lower, such as 25 % or lower, such as 20 % or lower, such as 15 % or lower, such as 10 % or lower, such as 5 % or lower, such as 2 % or lower, such as 1 % or lower.

In other preferred embodiments, the reference value of step c) may be a nuclear fraction of 0.5 - 30 %, such as 1 - 25 %, such as 5 - 15 %, such as 8 - 12%, such as 10 %.

Further, in embodiments of the methods of the above aspects, the criterion for the conclusion in step d) may be a sample value for staining intensity of a sample, i.e., a nuclear intensity, which is higher than absent nuclear intensity, such as higher than weak nuclear intensity, such as higher than moderate nuclear intensity. In alternative or complementing embodiments of the methods of the above aspects, the reference value of step c) may be an moderate nuclear intensity of ANLN protein expression or lower, such as a weak nuclear intensity of ANLN protein expression or lower, such as an absent nuclear intensity of ANLN protein expression.

Further, in embodiments of the methods of the above aspects, the reference value may be constituted of two values, wherein the criterion for the conclusion in step d) is a sample value being higher than any one of these two values.

Alternatively, in embodiments of the methods of the above aspects, the reference value may be a combination of a fraction value and an intensity value, such as a nuclear fraction value and a nuclear intensity value.

Also, in embodiments of the methods of the above aspects, the reference value may be a function of a nuclear fraction value and a nuclear intensity value. For example, such a function may be a staining score. The "staining score" is calculated as described in Examples, Section 3 and Table 1 below. For example, the reference value may be a staining score of 0, 1 or 2.

The person skilled in the art realizes that other reference values being an intensity value or a fraction value also fall within the scope of the present invention. Likewise, the person skilled in the art realizes that other combinations of fractions and intensities also fall within the scope of the present invention. Consequently, the reference value may involve two, and possibly even more, criteria.

In general, the selection of a nuclear intensity value and/or a nuclear fraction value as the reference value may depend on the staining procedure, e.g., on the employed anti-ANLN protein antibody and on the staining reagents.

Guided by the present disclosure, a person skilled in the art, e.g., a pathologist, understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of ANLN protein for establishing the appearance of a certain fraction or intensity.

However, a reference sample may not only be used for the provision of the actual reference value, but also for the provision of an example of a sample with an amount of ANLN protein that is higher than the amount corresponding to the reference value. As an example, in histochemical staining, such as in immunohistochemical staining, the skilled artisan may use a reference sample for establishing the appearance of a stained sample having a high amount of ANLN protein, e.g., a positive reference. Subsequently, the skilled artisan may assess the appearances of samples having lower amounts of ANLN protein, such as the appearance of a sample with an amount of ANLN protein corresponding to the reference value. In other words, the skilled artisan may use a reference sample to create a mental image of a reference value corresponding to an amount of ANLN protein which is lower than that of the reference sample. Alternatively, or as a complement, in such assessments, the skilled artisan may use another reference sample having a low amount of ANLN protein, or essentially lacking ANLN protein, for establishing the appearance of such sample, e.g., as a "negative reference".

For example, if a reference value of 10 % nuclear fraction is used, the kit may comprise a first reference sample having no detectable ANLN protein, and thus corresponding to a nuclear fraction of 0, which is lower than the reference value, and a second reference sample having an amount of ANLN protein corresponding to a nuclear fraction of 75 % or higher, which is higher than the reference value.

Consequently, in the evaluation, the skilled artisan may use a reference sample for establishing the appearance of a sample with a high amount of ANLN protein. Such reference sample may be a sample comprising tissue expressing a high amount of ANLN protein, such as a sample comprising breast tumor tissue having a pre-established high expression of ANLN protein.

Accordingly, the reference sample may provide an example of a strong nuclear intensity (ni). With the knowledge of the appearance of a sample with strong ni, the skilled artisan may then divide samples into other ni categories, such as the above-mentioned absent, weak, moderate and strong. This division may be further assisted by a reference sample lacking detectable ANLN protein (negative reference), i.e., a reference sample providing an absent nuclear intensity. Also, the reference sample may provide an example of a sample with a nuclear fraction (nf) of 75 % or higher. With the knowledge of the appearance of a sample with more than 75 % positive cells, the skilled artisan may then evaluate the nuclear fraction of other samples having e.g., a lower percentage of positive cells. This division may be further assisted by a reference sample essentially lacking ANLN protein (negative reference), i.e., a reference sample providing a low nf (such as ≤ 1 %), or a nf of 0.

As mentioned above, cell lines expressing a controlled amount of ANLN protein may be used as the reference, in particular as a positive reference.

As discussed above, the methods the third and fourth aspects may be adapted to a selected reference value, such as one of the reference values presented above, and the reference prognosis will in such case be a consequence of the selected reference value. As a non-limiting example, if an nuclear fraction of 1 % (nf = 1 %) is used as the reference value, an associated reference prognosis may be derived from figure 1 a (RFS analyzed) by looking at the "low" curve (solid line). At a given time from diagnosis, the cumulative survival corresponding to the "low" curve may be read from the figure, e.g., 92 % after 5 years (60 months), which results in a reference prognosis being a probability of five-year recurrence-free survival of 92 %. Consequently, subjects of the same group having sample values which are higher than the reference value nf = 1 % have a probability of five year recurrence-free survival which is lower than 92 %. Using the same logic, the associated reference prognosis may be a probability of five-year recurrence-free survival of lower than 84 % if using a nuclear fraction of 10 % as the reference value (Fig 1B).

In the latter example, wherein a nuclear fraction of 10 % is used as the reference value, the reference prognosis is based on subjects exhibiting a nf of 0 - 10 %, i.e., the reference prognosis is not directly derived from a group of subjects exhibiting the exact reference value. However, the conclusion that the subjects having a sample value which is higher than such reference value have a prognosis which is worse than such reference prognosis is still true.

Consequently, in embodiments of the methods of the above aspects, the reference prognosis may be a likelihood of five-year recurrence-free survival of 82 - 100 %, such as 87 - 97 %, if the reference value nf = 1 % is used. Further, in embodiments of the methods of the above aspects, the reference prognosis may be a likelihood of five-year recurrence-free survival of 74 - 94 %, such as 79 - 89 %, if the reference value nf = 10 % is used.

Also, in embodiments of the methods of the above aspects, the reference prognosis may be a five-year recurrence free survival of 72 % or higher if the reference value nf = 1 % is used (derivable from Fig 1a, "high" curve), a five-year recurrence free survival of 62 % or higher if the reference value nf = 10 % is used (derivable from Fig 1b, "high" curve) or a five-year recurrence free survival of 60 % or higher if the reference value nf = 25 % is used (derivable from Fig 1c, "high" curve).

Further, in embodiments of the methods of the above aspects, the reference prognosis may be a five-year breast cancer specific survival of 85 % or higher if the reference value nf = 1 % is used (derivable from Fig 3a, "high" curve) or five-year overall survival of 70 % or higher if the reference value nf = 1 % is used (derivable from Fig 4a, "high" curve).

However, the above reference prognoses are only provided as illustrative examples, and the skilled person understands that the usefulness of the methods according to the above aspects is not limited to any specific reference prognosis or prognosis measure.

Further, the skilled person should recognize that the usefulness of the methods according to the above aspects is not limited to the quantification of any particular variant of the ANLN protein present in the subject in question, as long as the protein is encoded by the relevant gene and presents the relevant pattern of expression. As a non-limiting example, the ANLN protein has an amino acid sequence which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the ANLN protein has an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acids may at the most be 85 % identical to a target sequence of 100 amino acids.

In embodiments of the methods of the aspects above, the ANLN protein may be detected and/or quantified through the application to the sample of a detectable and/or quantifiable affinity ligand, which is capable of selective interaction with the ANLN protein. The application of the affinity ligand is performed under conditions that enable binding of the affinity ligand to any ANLN protein in the sample.

To concretize, in embodiments of the methods of the aspects above, step b) may comprise:
b1) applying to the sample a quantifiable affinity ligand capable of selective interaction with the ANLN protein to be evaluated, the application being performed under conditions that enable binding of the affinity ligand to any ANLN protein present in the sample;
b2) removing non-bound affinity ligand; and
b3) quantifying any affinity ligand remaining in association with the sample to evaluate the amount.

"Affinity ligand remaining in association with the sample" refers to affinity ligand which was not removed in step b2), e.g., the affinity ligand bound to the sample. Here, the binding may for example be the interaction between antibody and antigen.

It is regarded as within the capabilities of those of ordinary skill in the art to select or manufacture the proper affinity ligand and to select the proper format and conditions for detection and/or quantification, once the connection between ANLN protein and a prognosis or treatment prediction for breast cancer is known through the teaching of the present disclosure. Nevertheless, examples of affinity ligands that may prove useful, as well as examples of formats and conditions for detection and/or quantification, are given below for the sake of illustration.

Thus, in some embodiments of the methods of the above aspects, an affinity ligand may be used, which is selected from the group consisting of antibodies, fragments thereof and derivatives thereof, i.e., affinity ligands based on an immunoglobulin scaffold. For example, the antibodies may be isolated and/or mono-specific. For example, antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. Monoclonal antibodies of defined specificity can be produced using the hybridoma technology developed by Köhler and Milstein (Köhler G and Milstein C (1976) Eur. J. Immunol. 6:511-519). The antibody fragments and derivatives of the present disclosure are capable of selective interaction with the same antigen (e.g. ANLN protein) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041). In embodiments of the methods of the above aspects, the quantifiable affinity ligand, e.g., an antibody or fragment thereof, may be obtainable by a process comprising a step of immunizing an animal with a protein whose amino acid sequence comprises the sequence SEQ ID NO:1, preferably with a protein consisting of the sequence SEQ ID NO:1.

Polyclonal and monoclonal antibodies, as well as their fragments and derivatives, represent the traditional choice of affinity ligands in applications requiring selective biomolecular recognition, such as in the detection and/or quantification of ANLN protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific and/or selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against ANLN protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); γ crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151; Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101 (25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the methods of the above aspects, an affinity ligand may be used, which is a non-immunoglobulin affinity ligand derived from any of the protein scaffolds listed above, or an oligonucleotide molecule.

The ANLN protein SEQ ID NO:1 was designed to consist of a unique sequence with low homology with other human proteins and to minimize cross reactivity of generated affinity reagents. Consequently, in embodiments of the methods of the above aspects, the quantifiable affinity ligand may be capable of selective interaction with an ANLN protein consisting of the sequence SEQ ID NO:1.

In some embodiments of the methods of the above aspects, an affinity ligand capable of selective interaction with the ANLN protein is detectable and/or quantifiable. The detection and/or quantification of such an affinity ligand may be accomplished in any way known to the skilled person for detection and/or quantification of binding reagents in assays based on biological interactions. Thus, any affinity ligand, as described above, may be used quantitatively or qualitatively to detect the presence of the ANLN protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with ANLN protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole) and bioluminescent proteins (e.g., luciferin, luciferase), haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g., ³H, ¹⁴C, ³²P, ³⁵S or ¹²⁵I) and particles (e.g., gold). In the context of the present disclosure, "particles" refer to particles, such as metal particles, suitable for labeling of molecules. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its ANLN protein target may involve visualizing techniques, such as light microscopy or immunofluoresence microscopy. Other methods may involve the detection via flow cytometry or luminometry.

A biological sample, such as a tumor tissue sample (biopsy), for example from breast tissue, which has been removed from the subject may be used for detection and/or quantification of ANLN protein. The biological sample, such as the biopsy, may be an earlier obtained sample. If using an earlier obtained sample in a method, no steps of the method are practiced on the human or animal body. The affinity ligand may be applied to the biological sample for detection and/or quantification of the ANLN protein. This procedure enables not only detection of ANLN protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from chemical reaction. Those of skill in the art are aware that a variety of different protocols can be modified in order for proper detection and/or quantification.

In embodiments of the methods of the above aspects, a biological sample may be immobilized onto a solid phase support or carrier, such as nitrocellulose or any other solid support matrix capable of immobilizing ANLN protein present in the biological sample applied to it. Some well-known solid state support materials useful in the present invention include glass, carbohydrate (e.g., Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand specific to ANLN protein may be applied, e.g., as described in Examples, Sections 3 or 6, of the present disclosure. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art should be able to determine operative and optimal assay conditions for each determination by routine experimentation.

Consequently, in embodiments of the methods of the above aspects, the quantifiable affinity ligand of b1) may be detected using a secondary affinity ligand capable of recognizing the quantifiable affinity ligand. The quantification of b3) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the ANLN protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the ANLN protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize ANLN protein by detection of radioactivity *in vivo* or *in vitro.* Radionuclear scanning with e.g., gamma camera, magnetic resonance spectroscopy or emission tomography function for detection *in vivo* and *in vitro,* while gamma/beta counters, scintillation counters and radiographies are also used *in vitro.*

As a sixth aspect of the present disclosure, there is provided a kit for carrying out a method according to an embodiment of the above aspects, which comprises:
a) a quantifiable affinity ligand capable of selective interaction with an ANLN protein; and
b) reagents necessary for quantifying the amount of the affinity ligand.

Various components of the kit according to the sixth aspect may be selected and specified as described above in connection with the method aspects of the present disclosure.

Thus, the kit according to the present disclosure comprises an affinity ligand against an ANLN protein, as well as other means that help to quantify the specific and/or selective affinity ligand after it has bound specifically and/or selectively to the ANLN protein. For example, the kit may contain a secondary affinity ligand for detecting and/or quantifying a complex formed by the ANLN protein and the affinity ligand capable of selective interaction with the ANLN protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g., endogenous enzyme block solution to decrease the background staining and/or counterstaining solution to increase staining contrast, that are commonly used in immunoassay reagent kits.

Thus, in embodiments of the kit aspect, the quantifiable affinity ligand is selected from the group consisting of antibodies, fragments thereof and derivatives thereof. As an example, such quantifiable affinity ligand may be obtainable a process comprising a step of immunizing an animal, such as a rabbit, with a protein whose amino acid sequence comprises the sequence SEQ ID NO:1 or a sequence which is at least 85 % identical to SEQ ID NO:1, preferably the sequence SEQ ID NO:1. Also, the quantifiable affinity ligand may be obtainable a process comprising a step of immunizing an animal, such as a rabbit, with a protein whose amino acid sequence consists of the sequence SEQ ID NO:1. Such immunization may for example be performed as described in Examples, Section 2, below. Also, the antibodies, fragments thereof and derivatives may for example be isolated and/or mono-specific. An example of an isolated and mono-specific polyclonal antibody, including the process of preparation thereof, is provided below in Examples, Section 2.

Alternatively, the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers. As a further alternative, the quantifiable affinity ligand is an oligonucleotide molecule.

In embodiments of the kit aspect, the quantifiable affinity ligand may be capable of selective interaction with an ANLN protein comprising a sequence selected from:
i) SEQ ID NO:2; and
ii) a sequence which is at least 85 % identical to SEQ ID NO: 2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

As further discussed above, the sequence SEQ ID NO:1 has been designed to be a particularly suitable antigen for being recognized by the affinity ligands of the present disclosure. Consequently, in embodiments of the kit aspect, the quantifiable affinity ligand may be capable of selective interaction with an ANLN protein comprising a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

Further, the ANLN protein with which the quantifiable affinity ligand may be capable of selective interaction, may consist of 200 amino acid residues or less, such as 190 amino acid residues or less, such as 180 amino acid residues or less, such as 170 amino acid residues or less, such as 160 amino acid residues or less, such as 150 amino acid residues or less, such as 140 amino acid residues or less, such as 136 amino acids.

SEQ ID NO:1 consists of 136 amino acids.

In embodiments of the kit aspect, the quantifiable affinity ligand may be capable of selective interaction with an ANLN protein consisting of the sequence SEQ ID NO:1.

Further, in embodiments of the kit aspect, the detectable affinity ligand may comprise a label selected from the group consisting of fluorescent dyes and metals, chromophoric dyes, chemiluminescent compounds and bioluminescent proteins, enzymes, radioisotopes, particles and quantum dots. Alternatively, the reagents necessary for quantifying the amount of the affinity ligand comprise a secondary affinity ligand capable of recognizing the quantifiable affinity ligand. As an example, the secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

The kit according to the kit aspect may also advantageously comprise a reference sample for provision of, or yielding, the reference value to be used for comparison with the sample value. For example, the reference may sample comprise a predetermined amount of ANLN protein. Such a reference sample may for example be constituted by a tissue sample having the predetermined amount of ANLN protein. The tissue reference sample may then be used by the person of skill in the art in the determination of the ANLN expression status in the sample being studied, by manual, such as ocular, or automated comparison of expression levels in the reference tissue sample and the subject sample. As another example, the reference sample may comprise cell lines, such as cancer cell lines, expressing a predetermined, or controlled, amount of ANLN protein. The person skilled in the art understands how to provide such cell lines, for example guided by the disclosure of Rhodes et al. (2006) The biomedical scientist, p 515-520. As an example, the cell lines may be formalin fixed. Also, such formalin fixed cell lines may be paraffin embedded.

The wording "reference sample for provision of the reference value" is to be interpreted broadly in the context of the present disclosure. The reference sample may comprise an amount of ANLN protein actually corresponding to the reference value, but it may also comprise an amount of ANLN protein corresponding to a value being higher than the reference value. In the latter case, the "high" value may be used by a person performing the method as an upper reference (positive reference) for assessing, e.g., the appearance of, a reference value which is lower than the "high" value. The person skilled in the art of immunohistochemistry understands how to do such an assessment. Further, as an alternative or a complementing example, the skilled person may use another reference sample comprising a low amount of ANLN protein for provision of a "low" value in such an assessment, e.g., as a negative reference. This is further discussed above in connection with the method aspects.

Consequently, in embodiments of the kit aspect, the reference sample may comprise an amount of ANLN protein corresponding to the reference value. As an example, the reference sample may comprise an amount of ANLN protein corresponding to a nuclear fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower.

As shown in the attached figures, in particular Fig 1 and 8, a cut-off of nf 1 %, 10 % or 25 %, provide relevant prognostic or treatment predictive information. A nf of 1 % or 10 %, especially 10 %, appears to be particularly promising cut-off:s.

Thus, in preferred embodiments, the reference sample may comprise an amount of ANLN protein corresponding to a nuclear fraction of 35 % or lower, such as 30 % or lower, such as 25 % or lower, such as 20 % or lower, such as 15 % or lower, such as 10 % or lower, such as 5 % or lower, such as 2 % or lower, such as 1 % or lower.

In other preferred embodiments, the reference sample may comprise an amount of ANLN protein corresponding to a nuclear fraction of 0.5 - 30 %, such as 1 - 25 %, such as 5 - 15 %, such as 8 - 12 %, such as 10 %.

Alternatively, or as a complement, the reference sample may comprise an amount of ANLN protein corresponding to an moderate nuclear intensity of ANLN protein expression or lower, such as a weak nuclear intensity of ANLN protein expression or lower, such as an absent nuclear intensity of ANLN protein expression.

Further, the reference sample may comprise an amount of ANLN protein corresponding a staining score of 0, 1, 2 or 3.

The provision of cytoplasmic fraction values or cytoplasmic intensity values is discussed above in connection with the method aspects.

Further, in alternative or complementing embodiments of the kit aspect, the kit may comprise a reference sample comprising an amount of ANLN protein corresponding to a value being higher than the reference value. In these embodiments, the reference sample may for example comprise an amount of ANLN protein corresponding to a nuclear fraction of 75 % or higher and/or a strong cytoplasmic intensity of nuclear expression.

In other alternative or complementing embodiments of the kit aspect, the kit may comprise a reference sample comprising an amount of ANLN protein corresponding to a value being lower than the reference value, e.g., an absent nuclear intensity and/or a nuclear fraction of ≤ 1 % ANLN protein positive cells, such as 0 % ANLN protein positive cells.

Also, in alternative or complementing embodiments of the kit aspect, the kit may comprise: a reference sample comprising an amount of ANLN protein corresponding to a predetermined reference value; a reference sample comprising an amount of ANLN protein corresponding to a value being higher than a predetermined reference value; and/or a reference sample comprising an amount of ANLN protein corresponding to a value being lower than a predetermined reference value.

Consequently, embodiments of the kit may comprise: a first reference sample comprising an amount of ANLN protein being higher than a predetermined reference value; and a second reference sample comprising an amount of ANLN protein being lower than the predetermined reference value.

In embodiments of the kit aspect, the reference sample may be a tissue sample, such as a tissue sample adapted to ocular or microscopic evaluation. As an example, the tissue reference sample may be fixated in paraffin or buffered formalin and/or histo-processed to µm-thin sections that are mounted on microscopic glass-slides. The tissue reference sample may be further adapted to staining with affinity ligands, such as antibodies, against an ANLN protein.

Consequently, in embodiments of the kit aspect, the reference sample may be adapted to directly, or indirectly, provide any relevant reference value, such as any one of the reference values discussed above.

Accordingly, further embodiments of the reference sample of the kit aspect are discussed above in connection with the reference values and reference samples of the method aspects.

As further discussed above, the combination of a level of ANLN protein expression and hormone receptor status may provide information relevant for drawing conclusions regarding a treatment prediction or a prognosis for a breast cancer subject.

Thus, the kit may also include means for establishing the hormone receptor status of a subject.

Consequently, in embodiments of the kit aspect, the kit may further comprise:
a') a quantifiable affinity ligand capable of selective interaction with the estrogen receptor and b') reagents necessary for quantifying the amount of such affinity ligand; and/or
a") a quantifiable affinity ligand capable of selective interaction with the progesterone receptor and b") reagents necessary for quantifying the amount of such affinity ligand.

The quantifiable affinity ligands of a') and a") are provided for the determination of the ER status and PR status, respectively.

The reagents of b), b') and b"), may be the same or different.

Quantifiable affinity ligands appropriate for steps a') and a"), respectively, are well known to the skilled person and commercially available.

Consequently, the kit may include means for provision of the hormonal status information necessary for drawing conclusions according to some embodiments of the above method aspect.

An ANLN protein may have many application in the context of breast cancer prognostics or treatment prediction. For example, the ANLN protein may be used in the production, selection or purification of a prognostic agent or a endocrine treatment predictive agent.

In the context of the present disclosure, a "prognostic agent" refers to an agent having at least one property being valuable in an establishment of a prognosis. For example the "prognostic agent" may be capable of selective interaction with a prognostic marker. Thus, the prognostic agent is for establishing a prognosis for a patient having a breast cancer.

In the context of the present disclosure, a "prognostic marker" refers to a something material which presence is of value in an establishment of a prognosis.

Further, in the context of the present disclosure, an "endocrine treatment predictive agent" refers to an agent having at least one property being valuable in an establishment of a treatment prediction for an endocrine treatment of a mammalian subject having a breast cancer. For example the "endocrine treatment predictive agent" may be capable of selective interaction with an endocrine treatment indicator.

In the context of the present disclosure, an "endocrine treatment indicator" refers to something material that indicates a suitability of an endocrine treatment, such as a responsiveness to an endocrine treatment. As an example, an "endocrine treatment indicator for a mammalian subject having a breast cancer" may give information or guidance about whether the subject, such as a human, should undergo an endocrine treatment or is likely to benefit from an endocrine treatment.

Also, as described herein, the presence of ANLN protein may be relevant for breast cancer prognostics and selection of a breast cancer treatment.

Further, to suit one or more of the above-mentioned uses, an ANLN protein of 136 amino acids (SEQ ID NO:1) was designed to consist of a unique sequence with low sequence similarity to other human proteins, to minimize unwanted cross reactivity of generated affinity reagents, and still be of a suitable size to allow formation of conformational epitopes and allow efficient expression in bacterial systems.

Thus, as a seventh aspect of the present disclosure, there is provided an ANLN protein consisting of 200 amino acid residues or less, which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

In embodiments of the seventh aspect, the ANLN protein consists of 190 amino acid residues or less, such as 180 amino acid residues or less, such as 170 amino acid residues or less, such as 160 amino acid residues or less, such as 150 amino acid residues or less, such as 140 amino acid residues or less, such as 138 amino acid residues or less, such as 136 amino acids.

In an embodiment of the seventh aspect, the ANLN protein consists of the amino acid sequence of SEQ ID NO:1.

Further, as an eighth aspect of the present disclosure, there is provided an use of an ANLN protein as an endocrine treatment indicator or a prognostic marker.

Traditionally, endocrine treatment, such as tamoxifen treatment, has mainly been given to subjects having hormone receptor positive, such as ER+ and/or PR+, breast cancers, and consequently, treatment prediction is particularly relevant for this group.

Thus, the endocrine treatment indicator may for example be for a mammalian subject having a ER positive breast cancer.

Further, the prognostic marker may for example be a prognostic marker for cancer, such as breast cancer.

Further, the prognostic marker may for example be an indicator of the probability of having a breast cancer metastasis, such as a distant metastasis.

The prognostic and treatment predictive relevance of ANLN protein found by the inventors entails the application of ANLN protein in various assays or other laboratory set-ups.

As a configuration of the eighth aspect, there is provided a use of an ANLN protein, or an antigenically active fragment thereof, for the production, selection or purification of a prognostic agent for establishing a prognosis for a patient having a breast cancer or an endocrine treatment predictive agent.

In the context of the present disclosure, an "antigenically active fragment" of an ANLN protein is a fragment of sufficient size to be useful for the generation of an affinity ligand, e.g., an antibody, which should interact with an ANLN protein comprising the fragment.

For example, the prognostic agent, or the endocrine treatment predictive agent, may be an affinity ligand capable of selective interaction with the ANLN protein. For example, the affinity ligand may preferably be capable of selective interaction with a protein consisting of the sequence SEQ ID NO:1. Also, the affinity ligand may preferably be an antibody or a fragment or a derivative thereof. Further, the antibody may for example be isolated and/or mono-specific.

Guided by the teachings of the present disclosure, the person skilled in the art understands how to use ANLN protein in the production, selection or purification of the prognostic agent. For example, the use according to the configuration of the eighth aspect may comprise affinity purification on a solid support onto which the ANLN protein has been immobilized. The solid support may for example be arranged in a column. Further, the use may comprise selection of affinity ligands having specificity for the ANLN protein using a solid support onto which the polypeptide has been immobilized. Such solid support may be 96 well plates, magnetic beads, agarose beads or sepharose beads. Further, the use may comprise analysis of affinity ligands on a soluble matrix, for example using a dextran matrix, or use in a surface plasmon resonance instrument, such as a Biacore™ instrument, wherein the analysis may for example comprise monitoring the affinity for the immobilized ANLN protein of a number of potential affinity ligands.

Also, for the production of the prognostic agent or the treatment predictive agent, the ANLN protein may be used in an immunization of an animal. This is further discussed above.

Such use may be involved in a method comprising the steps:
i) immunizing an animal using the ANLN protein as the antigen;
ii) obtaining serum comprising the prognostic agent from the immunized animal; and, optionally,
iii) isolating the prognostic agent from the serum.
Alternatively the steps following the first step may be:
ii') obtaining cells from the immunized animal, which cells comprise DNA encoding the prognostic agent,
iii') fusing the cells with myeloma cells to obtain at least one clone, and
iv') obtaining the prognostic agent expressed by the clone.

In embodiments of the eighth aspect, the amino acid sequence of the ANLN protein may comprise a sequence selected from:
i) SEQ ID NO:1; and
ii) a sequence which is at least 85 % identical to SEQ ID NO:1.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

Further, in embodiments of the eighth aspect the amino acid sequence of the ANLN protein may comprise a sequence selected from:
i) SEQ ID NO:2; and
ii) a sequence which is at least 85 % identical to SEQ ID NO:2.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

Also, in embodiments of the eighth aspect, the ANLN protein may be any ANLN protein according to the seventh aspect.

The inventors have found that ANLN protein may be utilized for breast cancer prognostics and treatment prediction. Further, an affinity ligand with affinity for the ANLN protein has been developed. However, the inventive scope is not limited to a single type of affinity ligand, and the inventive idea may be put into practice using any type of affinity ligand capable of selective interaction with an ANLN protein.

Thus, as a ninth aspect of the present disclosure, there is provided an affinity ligand capable of selective interaction with an ANLN protein.

The ANLN protein SEQ ID NO:1 was designed to consist of a unique sequence with low homology with other human proteins and to minimize cross reactivity of generated affinity reagents. Thus, in an embodiment of the ninth aspect, the affinity ligand may be capable of selective interaction with an ANLN protein comprising, preferably consisting of, the amino acid sequence SEQ ID NO:1.

In an embodiment of the ninth aspect, the affinity ligand is an antibody or a fragment or a derivative thereof. For example, the antibody or a fragment or a derivative thereof may be isolated and/or mono-specific.

SEQ ID NO:1 was also especially designed for immunizations, e.g., designed to lack transmembrane regions to ensure efficient expression in E. coli, and to lack any signal peptide, since those are cleaved off in the mature protein. Consequently, the antibody, or fragment or derivative thereof, may for example be one that is obtainable by a process comprising a step of immunizing an animal, such as a rabbit, with a protein whose amino acid sequence comprises, preferably consists of, the sequence SEQ ID NO:1. For example, the immunization process may comprise primary immunization with the protein in Freund's complete adjuvant. Also, the immunization process may further comprise boosting at least two times, in intervals of 2-6 weeks, with the protein in Freund's incomplete adjuvant. Processes for the production of antibodies or fragments or derivatives thereof against a given target are known in the art, and may be applied in connection with this aspect of the present disclosure. Any of those variants of the ANLN protein (e.g., SEQ ID NO:2) or the antigenically active fragment thereof (e.g., SEQ ID NO:1) that are discussed above may, of course, be used in such a process for generating an antibody or a fragment or derivative thereof.

The affinity ligand may also be used for *in vivo* diagnosis, such as *in vivo* imaging.

Thus, in an embodiment of the ninth aspect, the affinity ligand is for use in an *in vivo* method for establishing a prognosis for a mammalian subject having a breast cancer or a prediction of an outcome of an endocrine treatment, such as a tamoxifen treatment, of a mammalian subject having a breast cancer, such as a ER positive breast cancer. The establishment of a prediction of the outcome of an endocrine treatment of a subject may for example be a determination of whether the subject is likely to benefit from the endocrine treatment. In such embodiments the affinity ligand may for example be labeled for enabling imaging, i.e. labeled with a detectable label. Appropriate labels for labeling affinity ligands, such as antibodies, are well known to the skilled person. *The in vivo* method for establishing a prognosis or a treatment prediction for a mammalian subject having a breast cancer may for example reveal ANLN protein expression in a tumor *in vivo,* which in turn may form the basis of a treatment decision. Various *in vivo* methods, labels and detection techniques that may be used in the context of this embodiment are further discussed above.

Further, in an embodiment of the ninth aspect, the affinity ligand may be for establishing a prognosis for a mammalian subject having a breast cancer. For example, the prognosis may be a probability of having a metastasis.

Also, in an embodiment of the ninth aspect, the affinity ligand may be for evaluating the amount of ANLN protein present in at least part of a sample from a subject having a breast cancer.

As a tenth aspect of the present disclosure, there is provided the use of an affinity ligand according to the ninth aspect as a prognostic agent.

In an embodiment of the tenth aspect, the use is for establishing a prognosis for a mammalian subject having a breast cancer. For example, the prognosis may be a probability of having a metastasis. The metastasis connection is further discussed above. Further,

As a configuration of the tenth aspect of the present disclosure, there is provided the use of an affinity ligand according to the ninth aspect as an endocrine treatment predictive agent.

In an embodiment of the configuration of the tenth aspect, the use is for establishing a prediction of an outcome of an endocrine treatment, such as a tamoxifen treatment, of a mammalian subject having a breast cancer, such as a ER positive breast cancer.

Also, there is provided the use of an affinity ligand according to the ninth aspect in the manufacture of a prognostic agent for the prognosis of a breast cancer or a endocrine treatment predictive agent.

In the light of the present disclosure, endocrine treatment, especially tamoxifen treatment, appears to primarily benefit those subjects who are hormone receptor positive and ANLN protein negative.

Thus, as a eleventh aspect of the present disclosure, there is provided an endocrine treatment product for use in the treatment of a mammalian subject, such as a human, having a breast cancer, wherein the subject is hormone receptor positive and ANLN protein negative.

As a related aspect thereof, there is provided a use of an endocrine treatment product in the manufacture of a medicament for treatment of a mammalian subject, such as a human, having a breast cancer, wherein the subject is hormone receptor positive and ANLN protein negative.

The subject being "hormone receptor positive" refers to that the breast cancer from the subject has been found to be positive for a hormone receptor, such as ER or PR, preferably ER. The person skilled in the art knows how to determine whether a breast cancer is positive for a hormone receptor or not. For example, a commercially available kit for determining ER or PR status may be used for the determination. The commercially available kit may for example be ER/PR pharmDX (DakoCytomation) and the skilled person may use the kit according to the manufacturers instructions.

Further, for establishing an ER or PR diagnosis, the skilled person may turn to Allred et al (1998) Mod Pathol 11(2), 155. Allred presents a total score (Allred score), and, for both ER and PR, an Allred score of higher than two is considered positive and an Allred score of two or lower is considered negative.

Alternatively, when classifying a sample as being hormone receptor positive or negative, such as ER+ or ER-, the skilled person may use 10 % positive cells as the cutoff, which is a recognized limit within the art.

In embodiments of the endocrine treatment product aspects, the hormone receptor positive breast cancer may be an ER+ or PR+ breast cancer, such as an ER+ breast cancer, a PR+ breast cancer, or an ER+ and PR+ breast cancer.

The subject being diagnosed as "ANLN protein negative" refers to that a tumor of a subject has been found negative for ANLN. For example, the subject may be considered ANLN protein negative if any ANLN protein parameter derived from the subject indicates that the subject is likely to benefit from an endocrine treatment. Further, the subject may be considered ANLN protein negative if a relevant biological sample from the subject has been found to contain an amount of ANLN protein corresponding to a sample value being lower than or equal to a relevant reference value. Relevant sample and reference values are discussed above in connection with the method aspects. As a non-limiting example, a breast cancer subject may be considered as ANLN protein negative if a relevant sample, such as a primary or secondary tumor sample, from the subject comprise an amount of ANLN protein which corresponds to a nuclear fraction of 25 % or lower, such as 20 % or lower, such as 15 % or lower, such as 10 % or lower, such as 5 % or lower, such as 1 % or lower, such as < 1%.

In embodiments of the endocrine treatment product aspects, the endocrine treatment product may be selected from a selective estrogen receptor modulator (SERM), an aromatase, and a steroidal estrogen receptor antagonist. The SERM may be e.g. toremifene, raloxifene, droloxifene, arzoxifene or tamoxifene. The aromatase inhibitor may be e.g. anastrozole, letrozole och exemestane. Further, the steroidal estrogen receptor antagonist may be fulvestrant. Preferably, the endocrine treatment product is a SERM, such as a SERM selected from toremifene, raloxifene, droloxifene, arzoxifene or tamoxifene. For example, in embodiments of the endocrine treatment product aspects, the endocrine treatment may be tamoxifen.

In all embodiments of all the above aspects involving an endocrine treatment, the endocrine treatment may be a SERM treatment, such as a tamoxifen treatment, e.g., of a mammalian subject having a hormone receptor positive, such ER positive and/or PR positive, breast cancer.

In the context of the present disclosure, "specific" or "selective" interaction of e.g., an affinity ligand with its target or antigen means that the interaction is such that a distinction between specific and non-specific, or between selective and non-selective, interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high specificity does not necessarily require high affinity. Molecules with low affinity (in the molar range) for its counterpart have been shown to be as specific as molecules with much higher affinity. In the case of the present disclosure, a specific or selective interaction refers to the extent to which a particular method can be used to determine the presence and/or amount of a specific protein, the target protein or a fragment thereof, under given conditions in the presence of other proteins in a tissue sample or fluid sample of a naturally occurring or processed biological fluid. In other words, specificity or selectivity is the capacity to distinguish between related proteins. Specific and selective are sometimes used interchangeably in the present description. For example, the specificity or selectivity of an antibody may be determined as in Examples, section 2, below, wherein analysis was performed using a protein array set-up and a Western blot. Specificity and selectivity determinations are also described in Nilsson P et al. (2005) Proteomics 5:4327-4337.

In the context of the present disclosure, a "mono-specific antibody" is one of a population of polyclonal antibodies which has been affinity purified on its own antigen, thereby separating such mono-specific antibodies from other antiserum proteins and non-specific antibodies. This affinity purification results in antibodies that bind selectively to its antigen. In the case of the present invention, the polyclonal antisera are purified by a two-step immunoaffinity based protocol to obtain mono-specific antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al. (2005) Proteomics 5:4327-4337).

### Examples

### Generation of mono-specific antibodies against ANLN protein and use thereof to detect ANLN protein in normal and cancerous samples

### 1. Generation of antigen

### a) Materials and methods

A suitable fragment of the target protein encoded by the EnsEMBL Gene ID ENSG00000011426 was selected using bioinformatic tools with the human genome sequence as template (Lindskog M et al. (2005) Biotechniques 38:723-727, EnsEMBL, www.ensembl.org). The fragment was used as template for the production of a 136 amino acid long fragment corresponding to amino acids 333-468 (SEQ ID NO:1) of the ANLN protein (SEQ ID NO:2; EnsEMBL entry no. ENSP00000265748). The protein fragment was designed to consist of a unique sequence with low sequence similarity to other human proteins, to minimize unwanted cross reactivity of generated affinity reagents, and still be of a suitable size to allow formation of conformational epitopes and allow efficient expression in bacterial systems.

A fragment of the ANLN gene transcript containing nucleotides 1201-1608 of EnsEMBL entry number ENST00000265748 (SEQ ID NO:3), was isolated by a Superscript™ One-Step RT-PCR amplification kit with Platinum® Taq (Invitrogen) and a human total RNA pool panel as template (Human Total RNA Panel IV, BD Biosciences Clontech). Flanking restriction sites Notl and Ascl were introduced into the fragment through the PCR amplification primers, to allow in-frame cloning into the expression vector (forward primer: ATTGTGAAGTCAACTTTATCCC, reverse primer: GTGAGTTTCCTGTTTGGTTTC). Resulting biotinylated PCR product was immobilized onto Dynabeads M280 Streptavidin (Dynal Biotech) (Larsson M et al (2000) J. Biotechnol. 80:143-157) and subjected to Not-Ascl digestion (New England Biolabs) on solid support by Notl-Ascl digestion, ligated into the pAff8c vector (Larsson M et al, supra) in frame with a N-terminal dual affinity tag consisting of a hexahistidyl tag for immobilized metal ion chromatography (IMAC) purification and an immunopotentiating albumin binding protein (ABP) from streptococcal protein G (Sjölander A et al (1997) J. Immunol. Methods 201:115-123; Ståhl S et al (1999) Encyclopedia of Bioprocess Technology: Fermentation, Biocatalysis and Bioseparation (Fleckinger MC and Drew SW, eds) John Wiley and Sons Inc., New York, pp 49-63), and transformed into E. coli BL21 (DE3) cells (Novagen). The sequences of the clones were verified by dye-terminator cycle sequencing of plasmid DNA amplified using TempliPhi DNA sequencing amplification kit (GE Healthcare, Uppsala, Sweden) according to the manufacturer's recommendations.

BL21 (DE3) cells harboring the expression vector were inoculated in 100 ml 30 g/l tryptic soy broth (Merck KGaA) supplemented with 5 g/l yeast extract (Merck KGaA) and 50 mg/l kanamycin (Sigma-Aldrich) by addition of 1 ml of an overnight culture in the same culture medium. The cell culture was incubated in a 1 liter shake flask at 37°C and 150 rpm until the optical density at 600 nm reached 0.5-1.5. Protein expression was then induced by addition of isopropyl-β-D-thiogalactopyranoside (Apollo Scientific) to a final concentration of 1 mM, and the incubation was continued overnight at 25°C and 150 rpm. The cells were harvested by centrifugation at 2400 *g*, and the pellet was re-suspended in 5 ml lysis buffer (7 M guanidine hydrochloride, 47 mM Na₂HPO₄, 2.65 mM NaH₂PO₄, 10 mM Tris-HCl, 100 mM NaCl, 20 mM β-mercaptoethanol; pH = 8.0) and incubated for 2 hours at 37 °C and 150 rpm. After centrifugation at 35300 g, the supernatant containing the denatured and solubilized protein was collected.

The His₆-tagged fusion protein was purified by immobilized metal ion affinity chromatography (IMAC) on columns with 1 ml Talon® metal (Co²⁺) affinity resin (BD Biosciences Clontech) using an automated protein purification procedure (Steen J et al. (2006) Protein Expr. Purif. 46:173-178) on an ASPEC XL4™ (Gilson). The resin was equilibrated with 20 ml denaturing washing buffer (6 M guanidine hydrochloride, 46.6 mM Na₂HPO₄, 3.4 mM NaH₂PO₄, 300 mM NaCl, pH 8.0-8.2). Clarified cell lysates were then added to the column. Thereafter, the resin was washed with a minimum of 31.5 ml washing buffer prior to elution in 2.5 ml elution buffer (6 M urea, 50 mM NaH₂PO₄, 100 mM NaCl, 30 mM acetic acid, 70 mM Na-acetate, pH 5.0). The eluted material was fractioned in three pools of 500, 700 and 1300 µl. The 700 µl fraction, containing the antigen, and the pooled 500 and 1300 µl fractions were stored for further use.

The antigen fraction was diluted to a final concentration of 1 M urea with phosphate buffered saline (PBS; 1.9 mM NaH₂PO₄, 8.1 mM Na₂HPO₄, 154 mM NaCl) followed by a concentration step to increase the protein concentration using Vivapore 10/20 ml concentrator with molecular weight cut off at 7500 Da (Vivascience AG). The protein concentration was determined using a bicinchoninic acid (BCA) micro assay protocol (Pierce) with a bovine serum albumin standard according to the manufacturer's recommendations. The protein quality was analyzed on a Bioanalyzer instrument using the Protein 50 or 200 assay (Agilent Technologies).

### b) Results

A gene fragment corresponding to nucleotides 1201-1608 of the long transcript (SEQ ID NO:3) of the ANLN gene and encoding a peptide (SEQ ID NO:1) consisting of amino acids 333 to 468 of the target protein ANLN (SEQ ID NO:2) was successfully isolated by RT-PCR from a human RNA pool using primers specific for the protein fragment. The 136 amino acid fragment (SEQ ID NO:1) of the target protein (SEQ ID NO:2) was designed to lack transmembrane regions to ensure efficient expression in *E. coli*, and to lack any signal peptide, since those are cleaved off in the mature protein. In addition, the protein fragment was designed to consist of a unique sequence with low homology with other human proteins, to minimize cross reactivity of generated affinity reagents, and to be of a suitable size to allow the formation of conformational epitopes and still allow efficient cloning and expression in bacterial systems.

A clone encoding the correct amino acid sequence was identified, and, upon expression in *E. coli*, a single protein of the correct size was produced and subsequently purified using immobilized metal ion chromatography. After dilution of the eluted sample to a final concentration of 1 M urea and concentration of the sample to 1 ml, the concentration of the protein fragment was determined to be 5.1 mg/ml and was 100 % pure according to purity analysis.

### 2. Generation of antibodies

### a) Materials and methods

The purified ANLN protein fragment as obtained above was used as antigen to immunize a rabbit in accordance with the national guidelines (Swedish permit no. A 84-02). The rabbit was immunized intramuscularly with 200 µg of antigen in Freund's complete adjuvant as the primary immunization, and boosted three times in four week intervals with 100 µg antigen in Freund's incomplete adjuvant.

Antiserum from the immunized animal was purified by a three-step immunoaffinity based protocol (Agaton C et al. (2004) J. Chromatogr. A 1043:33-40; Nilsson P et al. (2005) Proteomics 5:4327-4337). In the first step, 7 ml of total antiserum was buffered with 10x PBS to a final concentration of 1x PBS (1.9 mM NaH₂PO₄, 8.1 mM Na₂HPO₄, 154 mM NaCl), filtered using a 0.45 µm pore-size filter (Acrodisc®, Life Science) and applied to an affinity column containing 5 ml N-hydroxysuccinimide-activated Sepharose™ 4 Fast Flow (GE Healthcare) coupled to the dual affinity tag protein His₆-ABP (a hexahistidyl tag and an albumin binding protein tag) expressed from the pAff8c vector and purified in the same way as described above for the antigen protein fragment. In the second step, the flow-through, depleted of antibodies against the dual affinity tag His₆-ABP, was loaded at a flow rate of 0.5 ml/min on a 1 ml Hi-Trap NHS-activated HP column (GE Healthcare) coupled with the ANLN protein fragment used as antigen for immunization (SEQ ID NO:1). The His₆-ABP protein and the protein fragment antigen were coupled to the NHS activated matrix as recommended by the manufacturer. Unbound material was washed away with 1x PBST (1x PBS, 0.1 % Tween20, pH 7.25), and captured antibodies were eluted using a low pH glycine buffer (0.2 M glycine, 1 mM EGTA, pH 2.5). The eluted antibody fraction was collected automatically, and loaded onto two 5 ml HiTrap™ desalting columns (GE Healthcare) connected in series for efficient buffer exchange in the third step. The second and third purification steps were run on the ÄKTAxpress™ platform (GE Healthcare). The antigen selective (mono-specific) antibodies (msAbs) were eluted with PBS buffer, supplemented with glycerol and NaN₃ to final concentrations of 40 % and 0.02 %, respectively, for long term storage at -20 °C (Nilsson P et al. (2005) Proteomics 5:4327-4337).

The specificity and selectivity of the affinity purified antibody fraction were analyzed by binding analysis against the antigen itself and against 383 other human protein fragments in a protein array set-up (Nilsson P et al. (2005) Proteomics 5:4327-4337). The protein fragments were diluted to 40 µg/ml in 0.1 M urea and 1x PBS (pH 7.4) and 50 µl of each were transferred to the wells of a 96-well spotting plate. The protein fragments were spotted in duplicate and immobilized onto epoxy slides (SuperEpoxy, TeleChem) using a pin-and-ring arrayer (Affymetrix 427). The slide was washed in 1x PBS (5 min) and the surface was then blocked (SuperBlock®, Pierce) for 30 minutes. An adhesive 16-well silicone mask (Schleicher & Schuell) was applied to the glass before the mono-specific antibodies were added (diluted 1:2000 in 1x PBST to appr. 50 ng/ml) and incubated on a shaker for 60 min. Affinity tag-specific IgY antibodies were co-incubated with the mono-specific antibodies in order to quantify the amount of protein in each spot. The slide was washed with 1x PBST and 1x PBS twice for 10 min each. Secondary antibodies (goat anti-rabbit antibody conjugated with Alexa 647 and goat anti-chicken antibody conjugated with Alexa 555, Molecular Probes) were diluted 1:60000 to 30 ng/ml in 1x PBST and incubated for 60 min. After the same washing procedure, as for the first incubation, the slide was spun dry and scanned (G2565BA array scanner, Agilent), thereafter images were quantified using image analysis software (GenePix 5.1, Axon Instruments).

In addition, the specificity and selectivity of the affinity-purified antibody were analyzed by Western blot. Western blot was performed by separation of total protein extracts from selected human cell lines on pre-cast 10-20 % SDS-PAGE gradient gels (Bio-Rad Laboratories) under reducing conditions, followed by electro-transfer to PVDF membranes (Bio-Rad Laboratories) according to the manufacturer's recommendations. The membranes were blocked (5 % dry milk, 1x TBST; 0.1 M Tris-HCl, 0.5 M NaCl, 0.1 % Tween20) for 1 h at room temperature, incubated with the primary affinity purified antibody (diluted 1:500 in blocking buffer) and washed in TBST. The secondary HRP-conjugated antibody (swine anti-rabbit immunoglobulin/HRP, DakoCytomation) was diluted 1:3000 in blocking buffer and chemiluminescence detection was carried out using a Chemidoc™ CCD camera (Bio-Rad Laboratories) and SuperSignal® West Dura Extended Duration substrate (Pierce), according to the manufacturer's protocol.

### b) Results

The quality of polyclonal antibody preparations has proven to be dependent on the degree of stringency in the antibody purifications, and it has previously been shown that depletion of antibodies directed against epitopes not originated from the target protein is necessary to avoid cross-reactivity to other proteins and background binding (Agaton C et al. (2004) J. Chromatogr. A 1043:33-40). Thus, a protein microarray analysis was performed to ensure that mono-specific polyclonal antibodies of high specificity had been generated by depletion of antibodies directed against the His₆-tag as well as of antibodies against the ABP-tag.

To quantify the amount of protein in each spot of the protein array, a two-color dye labeling system was used, with a combination of primary and secondary antibodies. Tag-specific IgY antibodies generated in hen were detected with a secondary goat anti-hen antibody labeled with Alexa 555 fluorescent dye. The specific binding of the rabbit msAb to its antigen on the array was detected with a fluorescently Alexa 647 labeled goat anti-rabbit antibody. The protein array analysis showed that the affinity purified mono-specific antibody against ANLN protein is highly selective to the correct protein fragment and has a very low background to all other protein fragments analyzed on the array.

The result of the Western blot analysis shows that the antibody specifically detects a single band of approximately 124 kDa in the neural stem-cell line RT-4 and in the Human Glioblastoma U-251 MG cell line. However, human plasma, liver and tonsil extracts did not express ANLN protein. The theoretical molecular weight of ANLN protein is 124kDa (as calculated from the ANLN protein amino acid sequence SEQ ID NO:2), corresponding well to the result obtained.

### 3. Tissue profiling by immunohistochemistry

### a) Material and methods

In total, 576 paraffin cores containing human tissues were analyzed using the mono-specific antibody sample obtained in Examples, section 2. All tissues used as donor blocks for tissue microarray (TMA) production were selected from the archives at the Department of Pathology, University Hospital, Uppsala, in agreement with approval from the local ethical committee. All tissue sections used for TMA analysis were examined to determine diagnosis and to select representative areas in donor blocks. Normal tissue was defined as microscopically normal (non-neoplastic) and was most often selected from specimens collected from the vicinity of surgically removed tumors. Cancer tissue was reviewed for diagnosis and classification. All tissues were formalin fixated, paraffin embedded, and sectioned for diagnostic purposes.

The TMA production was performed essentially as previously described (Kononen J et al. (1998) Nature Med. 4:844-847; Kallioniemi OP et al. (2001) Hum. Mol. Genet. 10:657-662). Briefly, a hole was made in the recipient TMA block and a cylindrical core tissue sample from the donor block was acquired and deposited in the recipient TMA block. This was repeated in an automated tissue arrayer from Beecher Instrument (ATA-27, Beecher Instruments, Sun Prairie, CA, USA) until a complete TMA design was produced. TMA recipient blocks were baked at 42 °C for 2 h prior to sectioning.

The design of TMA:s was focused on obtaining samples from a large range of representative normal tissues, and on including representative cancer tissues. This has previously been described in detail in Kampf C et al. (2004) Clin. Proteomics 1:285-300. In brief, samples from 48 normal tissues and from 20 of the most common cancer types affecting humans were selected. In total, eight different designs of TMA blocks, each containing 72 cores of tissue with 1 mm diameter, were produced. Two of the TMA:s represented normal tissues, corresponding to 48 different normal tissues in triplicates from different individuals. The remaining 6 TMA:s represented cancer tissue from 20 different types of cancer. For 17 of the 20 cancer types, 12 individually different tumors were sampled, and for the remaining 3 cancer types, 4 individually different tumors were sampled, all in duplicates from the same tumor. The TMA blocks were sectioned with 4 µm thickness using a waterfall microtome (Leica), and placed onto SuperFrost® (Roche Applied Science) glass slides for IHC analysis.

Automated IHC was performed as previously described (Kampf C et al. (2004) Clin. Proteomics 1:285-300). In brief, the glass slides were incubated for 45 min in 60 °C, de-paraffinized in xylene (2 x 15 min) and hydrated in graded alcohols. For antigen retrieval, slides were immersed in TRS (Target Retrieval Solution, pH 6.0, DakoCytomation) and boiled for 4 min at 125 °C in a Decloaking chamber® (Biocare Medical). Slides were placed in the Autostainer® (DakoCytomation) and endogenous peroxidase was initially blocked with H₂O₂ (DakoCytomation). The slides were incubated for 30 min at room temperature with the primary antibody obtained as in Examples, Section 2, followed by incubation for 30 min at room temperature with goat anti-rabbit peroxidase conjugated Envision®. Between all steps, slides were rinsed in wash buffer (DakoCytomation). Finally, diaminobenzidine (DakoCytomation) was used as chromogen and Harris hematoxylin (Sigma-Aldrich) was used for counterstaining. The slides were mounted with Pertex® (Histolab).

All immunohistochemically stained sections from the eight different TMA:s were scanned using a ScanScope T2 automated slide-scanning systems (Aperio Technologies). In order to represent the total content of the eight TMA:s, 576 digital images were generated. Scanning was performed at 20 times magnification. Digital images were separated and extracted as individual tagged image file format (TIFF) files for storage of original data. In order to be able to handle the images in a web-based annotation system, the individual images were compressed from TIFF format into JPEG format. All images of immunohistochemically stained tissue were manually evaluated under the microscope and annotated by a certified pathologist or by specially educated personnel, subsequently verified by a pathologist.

Annotation of each different normal and cancer tissue was performed using a simplified scheme for classification of IHC outcome. Each tissue was examined for representativity and immunoreactivity. The different tissue specific cell types included in each normal tissue type were annotated. For each cancer, tumor cells and stroma were annotated. Basic annotation parameters included an evaluation of i) subcellular localization (nuclear and/or cytoplasmic/membranous), ii) staining intensity (SI) and iii) fraction of stained cells (FSC). Staining intensity was subjectively evaluated in accordance to standards used in clinical histo-pathological diagnostics and outcome was classified as: absent = no immunoreactivity, weak = faint immunoreactivity, moderate = medium immunoreactivity or strong = distinct and strong immunoreactivity. The fraction of stained cells was estimated and classified as ≤ 1 %, 2 - 25 %, 26 - 75 % or > 75 % immunoreactive cells of the relevant cell population. Based on both the intensity and fraction of immunoreactive cells, a "staining score" was given for each tissue sample: 0 = negative, 1 = weak, 2 = moderate and 3 = strong. N.R. means that no representative tissues were present. In detail, the staining score was given according to the following criteria: 0 was given if SI = absent or weak and FSC ≤ 25%; 1 was given if SI = weak and FSC > 25% or if SI = moderate and FSC ≤ 25%; 2 was given if SI = moderate and FSC > 25% or if SI = strong and FSC ≤ 25% and finally 3 was given if SI = strong and FSC > 25%, see also table 1. The skilled artisan should recognize that this procedure is similar to a calculation of an Allred score, see e.g., Allred et al. (1998) Mod Pathol 11 (2), 155.

| **Table 1:** Staining score | | |
|---|---|---|
| **Staining score** | **Staining intensity** | **Fraction of stained cells** |
| 0 | absent | N.A. |
| 0 | absent | N.A |
| 0 | absent | N.A |
| 0 | absent | N.A |
| 0 | weak | ≤ 1% |
| 0 | weak | 2 - 25% |
| 1 | weak | 26 - 75% |
| 1 | weak | > 75% |
| 1 | moderate | ≤ 1% |
| 1 | moderate | 2 - 25% |
| 2 | moderate | 26 - 75% |
| 2 | moderate | > 75% |
| 2 | strong | ≤ 1% |
| 2 | strong | 2 - 25% |
| 3 | strong | 26 - 75% |
| 3 | strong | > 75% |

### b) Results

The results from tissue profiling with the mono-specific antibody generated towards a recombinant protein fragment of the human target protein ANLN obtained as in Examples, Section 2 showed a particular immunoreactivity in several normal tissues. Table 2 shows the ANLN protein expression pattern in normal human tissues. Using IHC and TMA technology, 144 spots (1 mm in diameter) representing 48 different types of normal tissue were screened for expression of ANLN protein. Immunoreactivity was observed in the vast majority of analyzed tissue samples. Strong expression was detected in the nucleus, but most samples exhibited additional weaker cytoplasmic and/or membranous staining. A staining score of 3 was found in several tissues. In a few cases no representative tissue (N.R.) were observed.

| **Table 2:** Expression pattern of ANLN protein in normal tissues | | |
|---|---|---|
| **Tissue type** | **Cell type** | **Staining score** |
| **Adrenal gland** | cortical cells | 1 |
| **Appendix** | glandular cells | 3 |
| | lymphoid tissue | 0 |
| **Bone marrow** | bone marrow poietic cells | 3 |
| **Breast** | glandular cells | 2 |
| **Bronchus** | respiratory epithelial cells | 2 |
| **Cerebellum** | cells in granular layer | 0 |
| | cells in molecular layer | 2 |
| | purkinje cells | 3 |
| **Cerebral cortex** | glial cells | 1 |
| | neuronal cells | 3 |
| **Cervix, uterine** | glandular cells | 1 |
| | squamous epithelial cells | 2 |
| **Colon** | glandular cells | 3 |
| **Corpus, uterine 1** | cells in endometrial stroma | 0 |
| | glandular cells | 2 |
| **Corpus, uterine 2** | cells in endometrial stroma | 1 |
| | glandular cells | 2 |
| **Duodenum** | glandular cells | 3 |
| **Epididymis** | glandular cells | 2 |
| **Esophagus** | squamous epithelial cells | 1 |
| **Fallopian tube** | glandular cells | 3 |
| **Gall bladder** | glandular cells | 2 |
| **Heart muscle** | myocytes | 2 |
| **Hippocampus** | glial cells | 1 |
| | neuronal cells | 3 |
| **Kidney** | cells in glomeruli | 0 |
| | cells in tubules | 2 |
| **Lateral ventricle** | glial cells | 1 |
| | neuronal cells | 3 |
| **Liver** | bile duct cells | 1 |
| | hepatocytes | 3 |
| **Lung** | alveolar cells | 1 |
| | macrophages | 2 |
| **Lymph node** | lymphoid cells outside reaction centra | 1 |
| | reaction center cells | 3 |
| **Nasopharynx** | respiratory epithelial cells | 1 |
| **Oral mucosa** | squamous epithelial cells | 2 |
| **Ovary** | follicle cells | N.R. |
| | ovarian stromal cells | 0 |
| **Pancreas** | exocrine glandular cells | 3 |
| | islet cells | 1 |
| **Parathyroid gland** | glandular cells | 2 |
| **Placenta** | decidual cells | N.R. |
| | trophoblastic cells | 3 |
| **Prostate** | glandular cells | 2 |
| **Rectum** | glandular cells | 3 |
| **Salivary gland** | glandular cells | 0 |
| **Seminal vesicle** | glandular cells | 2 |
| **Skeletal muscle** | myocytes | 1 |
| **Skin** | adnexal cells | N.R. |
| | epidermal cells | 2 |
| **Small intestine** | glandular cells | 3 |
| **Smooth muscle** | smooth muscle cells | 1 |
| **Soft tissue 1** | mesenchymal cells | 2 |
| **Soft tissue 2** | mesenchymal cells | 2 |
| **Spleen** | cells in red pulp | 1 |
| | cells in white pulp | 0 |
| **Stomach 1** | glandular cells | 3 |
| **Stomach 2** | glandular cells | 3 |
| **Testis** | cells in seminiferus ducts | 3 |
| | leydig cells | 2 |
| **Thyroid gland** | glandular cells | 3 |
| **Tonsil** | lymphoid cells outside reaction centra | 1 |
| | reaction center cells | 3 |
| | squamous epithelial cells | 2 |
| **Urinary bladder** | urothelial cells | 2 |
| **Vagina** | squamous epithelial cells | 2 |
| **Vulva/anal skin** | squamous epithelial cells | 2 |

ANLN protein expression was further evaluated in tissue samples from various cancer types. Table 3 shows the level of ANLN protein expression in 12 different breast carcinoma tissues samples. 11 of these samples showed representative tissue and all of these showed positivity, i.e., a staining score of higher than zero. ANLN protein expression was observed in both the nucleus and cytoplasm with the strongest expression in the nucleus.

| **Table 3:** Expression pattern of ANLN protein in breast carcioma tissues | | | | | | | | | | | | |
|---|---|---|---|---|---|---|---|---|---|---|---|---|
| **Tissue sample** | 1 | 2 | 3 | 4 | 5 | 6 | 7 | 8 | 9 | 10 | 11 | 12 |
| **Staining score** | 3 | 3 | 3 | 3 | 3 | 2 | 2 | 2 | 2 | 2 | 1 | N.R. |

### 4. Consecutive cohort TMA (Cohort I)

### a) Material and methods

Archival formalin-fixed paraffin-embedded tissue from 498 patients diagnosed with primary breast cancer between 1988 and 1992 was collected from the Department of Pathology, Malmö University Hospital, Sweden. The median age of patients was 65 (range 27-96) years and median follow-up time was 128 months (0-207). Information regarding the date of death was obtained from the regional cause-of-death registries for all patients. Ethical permission was obtained from the Local Ethics Committee of Lund University.

All 498 cases of breast cancer were histopathologically reevaluated on slides stained with hematoxylin and eosin. TMA:s were constructed by sampling 2 x 1.0 mm cores per case from areas representative of invasive cancer, using a manual arraying device (ATA-27, Beecher Inc, Sun Prairie, WI, USA). The TMA:s were prepared and automated IHC was performed as described in section 3 above, using the ANLN protein antibody prepared as described in section 2 above. IHC staining of ER had been performed previously. In line with current clinical praxis, a cut-off at 10 % positive nuclei was used to define hormone receptor positivity.

For statistical analyses, the nucleic fraction (nf) level was evaluated, in line with what is described in Examples, Section 3 above. The nuclear fraction was subjectively evaluated in accordance to standards used in clinical histopathological diagnostics and outcome was classified as ≤ 1 %, 2 - 25 %, 26 - 75 % or > 75 %. Based on the survival trends for all different strata, dichotomized variables were constructed for further statistical analyses. For this purpose the 2-25% group was split into two groups namely 2-10% and >10-25%. Subsequently, groups denoted "high" or "low" were established based on ANLN protein expression. In a first analysis, the "high" group represented a nuclear fraction (nf) of > 1%, and the "low" group a nuclear fraction ≤ 1 %. In a second analysis, the "high" group represented a nuclear fraction (nf) of > 10%, and the "low" group a nuclear fraction ≤ 10 %. In a third analysis, the "high" group represented a nuclear fraction (nf) of > 25%, and the "low" group a nuclear fraction ≤ 25 %. This classification of samples was used for OS, RFS and BCSS analysis according to the Kaplan-Meier estimator, and the log-rank test was used to compare survival in different strata. All statistical tests were two-sided, and p-values of < 0.05 were considered significant. All calculations were made with the statistical package SPSS 12.0 (SPSS Inc. Illinois, USA).

### b) Results

Immunohistochemistry analysis of ANLN protein expression could be performed on 467 of 498 tumor samples and 400 of these were identified as ER positive. The remaining cores either did not contain invasive cancer or had been lost during histoprocessing. An initial survival analysis to define an optimal cut off for high vs. low level of ANLN protein was done (Fig 1). This analysis revealed that significant difference was obtained at a cut off of 1 % (p=0.000), 10% (p=0.001) as well as at 25% (p=0.015). 1 % cut off was chosen for further statistical analyses because it yielded the most significant result. Figure 2, 3 and 4 show survival analysis based on high and low ANLN protein level using the 1% cut off level. Figure 2a shows that the RFS for all subjects are significantly better for ANLN protein low patients than for ANLN protein high patients. In figure 2b ER positive subjects were analyzed in the same way as in 2a and again a significantly better survival was observed for ANLN protein low patients. BCSS and OS also showed similar significant results, which may be seen in figure 3 and 4.

Taken together, this indicates that a prognostic relevance of ANLN protein expression over a range of cut-off values, particularly nuclear fraction cut-off values of 25 % or lower. Further, the figures shows that analysis using different survival measures yields prognostically relevant results. RFS appears to be particularly promising.

The ANLN protein level was found to correlate with metastasizing activity. Figure 5 shows a higher frequency of metastases in subjects with high ANLN protein level as compared to subjects with low ANLN protein level. This shows that high ANLN protein expression indicates a high probability of having a metastasis.

Further, probability of recurrence free survival for ANLN protein low subjects treated with tamoxifen was found to be significantly better than for the ANLN protein high patients (Fig 6, p=0.018), indicating the prognostic relevance of ANLN protein expression for breast cancer patient treated with tamoxifen. Further, this suggests a treatment predictive role of ANLN protein.

### 5. Randomized premenopausal cohort TMA (Cohort II)

### a) Material and methods

Immunohistochemistry analyses were performed on TMAs representing 500 tumors from 564 premenopausal patients enrolled in a controlled, randomized tamoxifen trial of stage II (pT2 N0 M0, pT1 N1 M0 or pT2 N1 M0) primary breast carcinoma. The patients had been included in the trial between 1986-1991 irrespective of hormone receptor status and 276 patients had been allocated to two years of adjuvant tamoxifen and 288 to control. Median age at diagnosis was 45 (25-57) years. Median follow-up was 13.9 years and less than 2% of the patients had received adjuvant chemotherapy. The study design is described in detail elsewhere (Rydén L et al, Eur J Cancer, 2005, 41 (2): 256-64). Ethical permission was obtained from the Local Ethics Committees at Lund and Linköping Universities.

All cases had been histopathologically re-evaluated on hematoxylin and eosin stained slides. TMA:s were constructed by sampling 2 x 0.6 mm cores per case from areas representative of invasive cancer, using an automated arraying device (ATA-27, Beecher Inc, Sun Prairie, WI, USA). The TMA:s were prepared and automated IHC was performed as described in section 3 above, using the ANLN protein antibody prepared as described in section 2 above. IHC staining of ER had been performed previously. In line with current clinical praxis, a cut-off at 10 % positive nuclei was used to define hormone receptor positivity.

For statistical analyses, categories were based on nuclear fraction (nf). nf was evaluated as in Examples, section 4, above. As with "the fraction of stained cells" in Examples, Section 3 above, nf was classified as ≤ 1 %, 2 - 25 %, 26 - 75 % or > 75 %. For this purpose the 2-25% group were split into two groups namely 2-10% and >10-25%. Based on the survival trends for all different strata, dichotomized variables were constructed for further statistical analyses. For this purpose the 2-25% group was split into two groups namely 2-10% and >10-25%. Subsequently, groups denoted "high" or "low" were established based on ANLN protein expression. In a first analysis, the "high" group represented a nuclear fraction (nf) of > 1 %, and the "low" group a nuclear fraction ≤ 1 %. In a second analysis, the "high" group represented a nuclear fraction (nf) of > 10%, and the "low" group a nuclear fraction ≤ 10 %. In a third analysis, the "high" group represented a nuclear fraction (nf) of 26%, and the "low" group a nuclear fraction ≤ 25 %. This classification of samples was used for OS, RFS and BCSS analysis according to the Kaplan-Meier estimator, and the log-rank test was used to compare survival in different strata. All statistical tests were two-sided, and p-values of < 0.05 % were considered significant. All calculations were made with the statistical package SPSS 16.0 (SPSS Inc. Illinois, USA).

### b) Results

Immunohistochemistry analysis of ANLN protein expression could be performed on 357 of 500 tumor samples. The remaining cores either did not contain invasive cancer or had been lost during histoprocessing. An initial survival analysis to define an optimal cut off for high vs. low level of ANLN protein was done (Fig 7). This analysis revealed that significant difference was obtained at a cut off of 10% (p=0.001). Cut off at 1 % and 25% showed similar trends.

Analysis of more clinically interesting groups from this randomized cohort is easily obtained by dividing tamoxifen treated and non-treated patients into separate groups. Figure 8 shows that RFS for ANLN protein low patients treated with tamoxifen is significantly better than for such ANLN protein high subjects. A cut off at both 1% and 10% gave significant p-values, whereas a cut off at 25% only revealed a trend. Similar results were obtained when analyzing BCSS and OS as seen in figure 9 and 10. Again a cut off at 10% were significant, whereas a trend were observed at 1 %.

If the parameters were changed, and the RFS analysis was based on ANLN protein level, the result revealed that tamoxifen treatment in subjects with high ANLN protein level had no positive impact on the outcome (Fig 11). Similar results were also obtained for BCSS and OS analysis (Fig 12 and 13). Even more interesting, the similar result was obtained for ER positive patients with high ANLN protein level, a group of patients that routinely has been prescribed tamoxifen (Fig 14b, 15b and 16b). Further, survival analysis (RFS, BCSS and OS) of the group of ER positive patients with invasive ductal carcinoma, one of the major invasive breast cancers, show similar result (Fig 17a-c). ER positive ANLN protein low patients on the other hand seem to, at least slightly, benefit from tamoxifen treatment (Fig 14a, 15a and 16a). Consequently, it may be concluded that patients with high level of ANLN protein, should not be recommended adjuvant tamoxifen treatment, and that an alternative treatment strategy should be considered for this group of patients. ANLN protein high patients have a overall lover survival probability as compared to ANLN protein low subjects. This is important information as ER positive patients generally is thought upon as a group with relatively good prognosis and with a rather straight forward treatment strategy. In particular for ER positive and ANLN protein high patients, this strategy may need to be reconsidered.

Further, in Fig 18 it is indicated that the findings with regard to the ER positive subgroup also applies to a subgroup of PR positive subjects.

### Establishment of a prognosis for a breast cancer patient

### 6. A non-limiting example

A breast cancer patient can present symptoms or signs such as a palpable lump/tumor, secretion from the mamilla or skin deformities. A proportion of breast cancers, generally without symptoms, are also detected by screening mammography.

Following the establishment of a breast cancer diagnosis in a patient, a tumor tissue sample is obtained. The tumor tissue sample may be obtained from a biopsy (a removal of a selected physical piece of tissue from the suspected tumor) performed earlier during the diagnosis of the cancer or from a specimen from an earlier surgical removal of the tumor. Further, for the provision of a "negative reference", a sample is taken from archival material comprising tissue lacking detectable ANLN protein expression. Such archival tissue may for example be breast cancer tissue having a pre-established low ANLN protein expression level or an appropriate tissue having a staining score of 0 in Table 2. Further, for the provision of a "positive reference", a sample is taken from archival material comprising tissue having high ANLN protein expression, such as breast cancer tissue having a pre-established high ANLN protein expression level or an appropriate tissue having a staining score of 3 in Table 2.

The sample material is fixated in buffered formalin and histo-processed in order to obtain thin sections (4 µm) of the of the sample material.

Immunohistochemistry is performed as described in Examples, Section 3. One or more sample sections from each sample are mounted on glass slides that are incubated for 45 min in 60 °C, de-paraffinized in xylene (2 x 15 min) and hydrated in graded alcohols. For antigen retrieval, slides are immersed in TRS (Target Retrieval Solution, pH 6.0, DakoCytomation) and boiled for 4 min at 125 °C in a Decloaking chamber® (Biocare Medical). Slides are placed in the Autostainer® (DakoCytomation) and endogenous peroxidase is initially blocked with H₂O₂ (DakoCytomation). The reason for mounting multiple sample sections may be to increase the accuracy of the results.

A primary ANLN protein specific antibody is added to the slides and incubated for 30 min in room temperature. The primary ANLN protein specific antibody may be obtained as in Examples, Section 2. Incubation of the primary ANLN protein specific antibody is followed by a 30 min incubation in room temperature with a labeled secondary antibody; e.g., goat-anti-rabbit peroxidase conjugated Envision®. To detect the secondary antibody, diaminobenzidine (DakoCytomation) is used as chromogen, contrasted with a Harris hematoxylin (Sigma-Aldrich) counterstaining. Between all steps, slides are rinsed in wash buffer (DakoCytomation). The slides are then mounted with Pertex® (Histolab) mounting media.

As a tool to validate the staining procedure, two control cell-lines may be used; e.g., one slide with cells expressing ANLN protein (positive cell line) and one slide having cells with no ANLN protein expression (negative cell line). The skilled artisan understands how to provide such cell lines, for example guided by the disclosure of Rhodes et al. (2006) The biomedical scientist, p 515-520. The control cell-line slides may be simultaneously stained in the same procedure as the breast cancer slides, i.e., incubated with the same primary and secondary antibodies.

To obtain digital images, the breast cancer tumor slides, the reference slides, and optionally, the slides with control cell-lines, may for example be scanned in a light microscope using a ScanScope T2 automated slide scanning system (Aperio Technologies) at x20 magnification. However, this scanning step is not necessary, but may make the procedure easier if, for example, the preparation and staining of the slides and the evaluation of the cytoplasmic intensity (see below) are performed at different locations or by different persons.

If control cell-lines are used, these are inspected to validate the staining procedure. If the cell-lines display staining results outside acceptable criteria, e.g., staining artifacts recognized by the skilled artisan, the staining of the biopsy samples is considered invalid and the whole staining procedure is repeated with new slides. If the positive and negative cell-lines display strong staining intensity and indistinct weak or no staining intensity, respectively, the staining is considered as valid.

The stained sample slide(s) from the tumor tissue biopsy is/are evaluated manually by visual inspection, and the nuclear fraction (nf) of the breast cancer slide(s) is/are graded as described in Examples, Section 4.

That is, the "nuclear fraction" (nf), which corresponds to the percentage of relevant cells in a sample that exhibits a positive staining in the nucleus, is subjectively evaluated in accordance to standards used in clinical histopathological diagnostics. A medium or distinct and strong immunoreactivity in the nucleus is considered positive and no or faint immunoreactivity in the nucleus is considered negative.

For example, a reference value of nf = 10 % may be selected due to its prognostic relevance, and in such case, the nf of the stained sample slide(s) from the tumor tissue biopsy is classified as ≤ 10 % or > 10 %. The person performing the evaluation and grading is aided by visual inspection of the stained reference slides: the reference slide having a high amount of ANLN protein provides a "positive reference", and the reference slide having no detectable ANLN protein expression provides a "negative reference". For example, the positive and negative references help the person performing the evaluation assessing the appearance of the reference value of nf 10 %, and thus, classifying the stained sample slide(s) from the tumor tissue biopsy as ≤ 10%or>10%.

If the stained sample slide(s) from the tumor tissue biopsy is/are classified as nf > 10 %, they are higher than the selected reference value of nf = 10 %, a conclusion is drawn that the prognosis is worse than a reference prognosis being associated with that reference value. For example, if looking at Fig 1b, the reference value of nf = 10 % is associated with a probability of five-year recurrence free survival of 85 % or higher. Consequently, in such case, the conclusion could be that the prognosis for the tested subject is worse than five-year recurrence free survival of 85 %.

### Establishment of a treatment prediction for an endocrine treatment of a breast cancer subject.

### 7. A non-limiting example

This example may be performed as in Examples, Section 6 until the conclusion is to be drawn. When establishing a treatment prediction for an endocrine treatment of a patient having breast cancer, it may be valuable to obtain the estrogen receptor status because traditionally, only patients having ER positive breast cancers are given endocrine treatment. Also, for this treatment prediction, a reference value of nf = 10 % may be used.

Consequently, the ER status of the patient is obtained before the conclusion is drawn. The obtained ER status may have been determined before or after the determination of the ANLN protein expression level. For example, the ER status may have been previously determined during the diagnosis of the cancer and/or determined from a specimen from an earlier surgical removal of the breast cancer tumor. The person skilled in the art knows how to determine whether a breast cancer is positive or negative for ER. For example, the commercially available kit ER/PR pharmDX (DakoCytomation) may be used for the determination. As another example, the method disclosed by Allred *et al.* (Allred et al. (1998) Mod Pathol 11(2), 155) may be used to obtain a total score (Allred score), and, for both ER and PR, an Allred score of higher than two is considered positive and an Allred score of two or lower is considered negative. Alternatively, when classifying a sample as being positive or negative for ER, a 10 % positive cells as the cut-off may be used, which is a recognized limit within the art.

If the breast cancer is ER positive and the stained sample slide(s) from the tumor tissue biopsy is/are classified as nf > 10 %, which is higher than the selected reference value of nf = 10 %, a conclusion is drawn that the patient is not likely to benefit from endocrine treatment. This is for example supported by Fig 14-16.

Consequently, a "high" value of ANLN protein is indicative of a non-response to an endocrine treatment, such as a tamoxifen treatment. The final decision to apply an endocrine treatment or not may depend on several parameters. However, a conclusion that the patient is ANLN protein "high" is in favor of a decision to refrain from treating the patient with an endocrine treatment, especially a tamoxifen treatment, even though the patient is ER positive.

All cited material, including but not limited to publications, DNA or protein data entries, and patents, referred to in this application are herein incorporated by reference.

The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. Method for determining whether a mammalian subject having a breast cancer is likely to benefit from an endocrine treatment, comprising the steps of:
a) providing a sample earlier obtained from said subject;
b) evaluating the amount of ANLN protein present in at least part of said sample, and determining a sample value corresponding to said amount;
c) comparing the sample value obtained in step b) with a reference value; and,
if said sample value is higher than said reference value,
d1) concluding that the subject is not likely to benefit from an endocrine treatment, or
if said sample value is lower than or equal to said reference value,
d2) concluding that the subject is likely to benefit from an endocrine treatment.

2. Non-treatment strategy method for a subject having a breast cancer, comprising:
a) providing a sample earlier obtained from the subject;
b) evaluating the amount of ANLN protein present in at least part of said sample, and determining a sample value corresponding to said evaluated amount;
c) comparing the sample value obtained in step b) with a reference value;
and, if said sample value is higher than said reference value,
d) refraining from treating said subject with an endocrine treatment.

3. Method for determining whether a prognosis for a mammalian subject having a breast cancer is worse than a reference prognosis, comprising the steps of:
a) providing a sample earlier obtained from said subject;
b) evaluating the amount of ANLN protein present in at least part of said sample, and determining a sample value corresponding to said evaluated amount;
c) comparing said sample value obtained in step b) with a reference value associated with said reference prognosis; and, if said sample value is higher than said reference value,
d) concluding that said prognosis for said subject is worse than said reference prognosis.

4. Method according to claim 3, wherein said prognosis is a probability of having a metastasis, said reference prognosis is a reference probability of having a metastasis and step d) comprises concluding that said probability is higher than said reference probability.

5. Method according to claim 3 or 4, wherein said subject has undergone, is undergoing and/or is scheduled for endocrine treatment.

6. Method according to any one of claims 1, 2 and 5, wherein said endocrine treatment is tamoxifen treatment.

7. Method according to any one of the preceding claims, wherein said breast cancer is hormone receptor positive, such as ER positive and/or PR positive.

8. Method according to any one of the preceding claims, wherein said sample is a breast cancer tissue sample.

9. Method according to any one of the preceding claims, wherein said evaluation of step b) is limited to the nucleus of tumor cells of said sample.

10. Method according to any one of the preceding claims, wherein said reference value is a nuclear fraction of 30 % ANLN protein positive cells, or lower.

11. Method according to any one of the preceding claims, wherein step b) comprises:
b1) applying to said sample a quantifiable affinity ligand capable of selective interaction with the ANLN protein to be evaluated, said application being performed under conditions that enable binding of said affinity ligand to any ANLN protein present in said sample;
b2) removing non-bound affinity ligand; and
b3) quantifying the affinity ligand remaining in association with said sample to evaluate said amount.

12. Kit for carrying out the method according to any one of the preceding claims, which comprises
a) a quantifiable affinity ligand capable of selective interaction with an ANLN protein; and
b) reagents necessary for quantifying the amount of said quantifiable affinity ligand.

13. Kit according to claim 12, further comprising at least one reference sample for provision of a reference value.

14. ANLN protein consisting of 200 amino acid residues or less, which comprises a sequence selected from:
i) SEQ ID NO:1; and
ii) a sequence which is at least 85 % identical to SEQ ID NO:1.

15. Affinity ligand capable of selective interaction with an ANLN protein.

16. Use *in vitro* of an affinity ligand according to claim 15 as a prognostic agent for establishing a prognosis for a mammalian subject having a breast cance or a endocrine treatment predictive agent for prediction of an outcome of an endocrine treatment of a mammalian subject having a breast cancer.

17. Endocrine treatment product for use in the treatment of a mammalian subject having a breast cancer, wherein said subject is hormone receptor positive, such as ER positive and/or PR positive, and ANLN protein negative.
